# EUROPÄISCHE PATENTANMELDUNG

(11) **EP 0 266 558 A2**
(43) Veröffentlichungstag der Anmeldung: **11.05.1988**
(21) Anmeldenummer: 87114316.0
(22) Anmeldetag: 01.10.1987
(51) Int. Cl.: C07D 401/04, C07D 403/04, C07D 401/06, C07D 409/04, C07D 417/04, C07D 235/08, C07D 235/18, C07D 235/16, C07D 235/30, C07D 405/04, C07D 235/06

(54) **5-Alkylbenzimidazole, Verfahren zu ihrer Herstellung und diese enthaltende Arzneimittel**

(30) Priorität: 07.10.1986 DE 3634066
(71) Anmelder: BOEHRINGER MANNHEIM GMBH, 68298 Mannheim (DE)
(72) Erfinder: Von der Saal, Wolfgang, Dr. rer. nat., D-6940 Weinheim (DE); Hölck, Jens-Peter, Dr. rer. nat., D-6800 Mannheim 31 (DE); Mertens, Alfred, Dr.rer.nat., D-6905 Schriesheim (DE); Müller-Beckmann, Bernd, Dr. med. vet., D-6718 Grünstadt (DE); Kling, Lothar, Dr., D-6800 Mannheim 31 (DE)

(57) **Zusammenfassung**

Verbindungen der Formel I 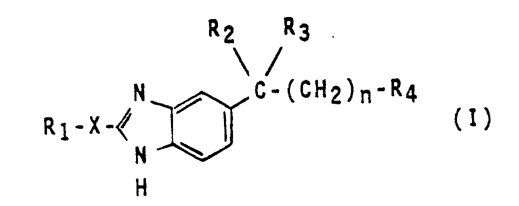 in der

R₁ einen gegebenenfalls substituierten Phenylring oder einen Naphtylrest, einen gegebenenfalls substituierten Heterocyclus,

oder für den Fall, daß X eine Bindung darstellt, R₁ neben den oben genannten Gruppen auch ein Wasserstoffatom, eine Alkyl-, Cycloalkyl-, Alkenyl-, Cycloalkenyl-, Alkinyl-, Halogenalkyl-, Alkoxyalkyl-, Carboxyalkyl-, Alkoxycarbonylalkyl-, Aminoalkyl-, eine cyclische Imino-, eine Hydroxy-, Mercapto-, Amino-, Alkylmercapto-, Alkylcarbonylamino-, Formylamino-, Alkylsulfonylamino-, Formylaminoalkyl-, Alkoxycarbonylaminoalkyl- oder eine Alkylsulfonylaminoalkylgruppe bedeutet,

R₂ und R₃ gleich oder verschieden sein können und Wasserstoff oder Alkylgruppen bedeuten, oder R₂ und R₃ zusammen mit dem C-Atom, an das sie gebunden sind, einen carbocyclischen Ring darstellen,

R₄ eine Cyano-, Aminocarbonyl-, Alkylaminocarbonyl-, Dialkylaminocarbonyl-, Hydrazinocarbonyl- oder Aminogruppe bedeutet, wobei die Hydrazinocarbonyl- oder die Aminogruppe gewünschtenfalls durch eine Formyl-, Alkylcarbonyl-, Trifluormethylcarbonyl-, Alkylsulfonyl-, Trifluormethylsulfonylgruppe, Phenylsulfonyl-, Alkylaminocarbonyloder Dialkylaminocarbonylgruppe substituiert sein kann,

X eine Bindung, eine Alkylen-, die Vinylen- oder die Iminogruppe -NH- bedeutet,

und

n einen Wert zwischen 0 und 5 annehmen kann,

deren Tautomere und deren physiologisch verträgliche Salze anorganischer oder organischer säuren, Verfahren zu ihrer Herstellung sowie diese Verbindungen enthaltende Arzneimittel zur Behandlung von Herz- und Kreislauferkrankungen.

## Beschreibung

Die vorliegende Erfindung betrifft neue 5-Alkylbenzimidazole der allgemeinen Formel I, 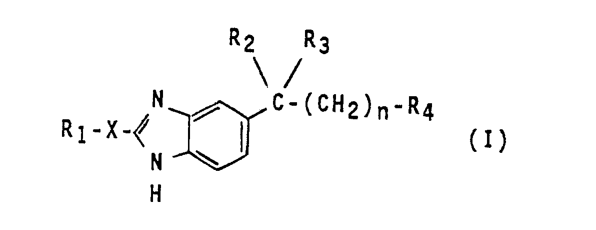
in welcher

R₁ einen Phenylring der allgemeinen Formel II darstellt, 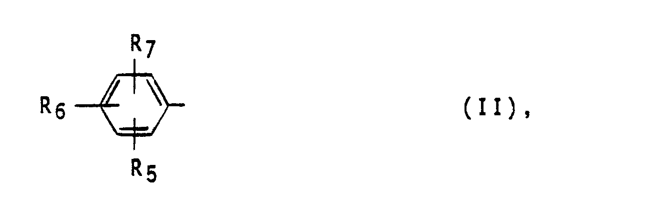
wobei R₅, R₆, R₇ gleich oder verschieden sein können und jeweils Wasserstoff, eine Alkansulfonyloxy-, Trifluormethansulfonyloxy-, Alkansulfonylamino-, Trifluormethansulfonylamino-, N-Alkyl-alkansulfonylamino-, N-Alkyltrifluormethansulfonylamino-, Alkylsulfenylmethyl-, Alkylsulfinylmethyl- oder Alkylsulfonylmethylgruppe, eine durch eine Hydroxy-, Alkoxy-, Amino-, Alkylamino- oder Dialkyl aminogruppe substituierte Carbonylgruppe, eine durch eine Amino-, Alkylamino-, Dialkylamino- oder cyclische Iminogruppe substituierte Sulfonylgruppe, wobei eine Methylengruppe in 4-Stellung durch ein Schwefel- oder Sauerstoffatom ersetzt sein kann, eine Alkylcarbonylamino-, Aminocarbonylamino- oder Alkylaminocarbonylaminogruppe, eine Alkylmercapto-, Alkylsulfinyl- oder Alkylsulfonylgruppe, eine Nitro-, Halogen-, Amino-, Hydroxy-, Alkyl-, Alkoxy-, Alkenyloxy-, Alkinyloxy-, Cyanalkyloxy-, Carboxyalkoxy-, Alkoxycarbonylalkyloxy-, Dialkylamino-, 1-Imidazolyl-, Trifluormethyl- oder Cyangruppe sein können,

oder einen Naphthylrest oder einen gesättigten oder ungesättigten heterocyclischen Funfring mit 1-4 Heteroatomen oder einen gesättigten oder ungesättigten heterocyclischen Sechsring mit 1-5 Heteroatomen darstellt, wobei die Heteroatome gleich oder verschieden sein können und Sauerstoff, Schwefel oder Stickstoff bedeuten und gewünschtenfalls an einem oder mehreren Stickstoffatomen ein Sauerstoffatom tragen können, und die Funf- oder Sechsringe gegebenenfalls durch eine oder mehrere Alkyl-, Alkoxy-, Alkylmercapto-, Hydroxy-, Nitro-, Amino-, Halogen- oder Cyangruppen substituiert oder mit einem Phenylring zu einem Bicyclus kondensiert sein können,

oder für den Fall, daß X eine Bindung darstellt, R₁ neben den oben genannten Gruppen auch ein Wasserstoffatom, eine Alkyl-, Cycloalkyl-, Alkenyl-, Cycloalkenyl-, Alkinyl-, Halogenalkyl-, Alkoxyalkyl-, Carboxylalkyl-, Alkoxycarbonylalkyl-, Aminoalkyl-, eine Hydroxy-, Mercapto-, Amino-, Alkylmercapto-, Alkylcarbonylamino-, Formylamino-, Alkylsulfonylamino-, Formylaminoalkyl-, Alkoxycarbonylaminoalkyl- oder eine Alkylsulfonylaminoalkylgruppe bedeutet,

R₂ und R₃ gleich oder verschieden sein können und Wasserstoff oder Alkylgruppen bedeuten, oder R₂ und R₃ zusammen mit dem C-Atom an das sie gebunden sind einen carbocyclischen Ring darstellen,

R₄ eine Cyano-, Aminocarbonyl-, Alkylaminocarbonyl-, Dialkylaminocarbonyl-, Hydrazinocarbonyl- oder Aminogruppe bedeutet, wobei die Hydrazinocarbonyl- oder die Aminogruppe gewünschtenfalls durch eine Formyl-, Alkylcarbonyl-, Trifluormethylcarbonyl-, Alkylsulfonyl-, Trifluormethylsulfonylgruppe, Phenylsulfonyl-, Alkylaminocarbonyl-oder Dialkylaminocarbonylgruppe substituiert sein kann,

X eine Bindung, eine Alkylen-, die Vinylen-, die Iminogruppe -NH- oder die Carbonylaminogruppe -CONH- bedeutet und

n einen Wert zwischen 0 und 5 annehmen kann,

deren Tautomere und deren physiologisch verträglichen Salze anorganischer und organischer Säuren und Verfahren zu ihrer Herstellung, sowie diese Verbindungen enthaltende Arzneimittel.

Für den Fall, daß Verbindungen der allgemeinen Formel I hergestellt wurden, die ein Asymmetriezentrum enthalten, sind auch Gegenstand der Erfindung die optisch aktiven Formen und racemischen Gemische dieser Verbindungen.

Diese neuen Verbindungen der vorliegenden Erfindung weisen wertvolle pharmakologische Eigenschaften auf, insbesondere bewirken sie an gehäuteten Herzmuskelfasern eine Steigerung der Ca²⁺-Sensibilitat und können damit Anwendung finden als Mittel zur Steigerung der Herzkraft. Weiterhin wirken sie blutdrucksenkend und/oder beeinflussen die Thrombozytenfunktion und verbessern die Mikrozirkulation.

Bedeutet R₁ einen Phenylring der allgemeinen Formel II, so kann der Alkylteil der bei R₅, R₆ und R₇ genannten Substituenten 1-5 Kohlenstoffatome enthalten, vorzugsweise 1-4 Kohlenstoffatome. Bevorzugt in diesem Sinne sind beispielsweise die Methansulfonyloxy-, Ethansulfonyloxy-, n-Propansulfonyloxy-, Isopropansulfonyloxy-, Trifluormethansulfonyloxy-, Methylsulfenylmethyl-, Ethylsulfenylmethyl-, n-Propylsulfenylmethyl-, Methylsulfinylmethyl-, Ethylsulfinylmethyl-, n-Propylsulfinylmethyl-, Methylsulfonylmethyl-, Ethylsulfonylmethyl-, n-Propylsulfonylmethyl-, Methansulfonylamino-, Ethansulfonylamino-, n-Propansulfonylamino-, Trifluormethansulfonylamino-, N-Methylmethansulfonylamino-, N-Ethyl-methansulfonylamino-, N-Methyl-ethansulfonylamino-, N-Ethyl-ethansulfonylamino-, N-Isopropyl-ethansulfonylamino-, N-Methyl-n-propansulfonylamino-, N-n-Propyl-n-propansulfonylamino-, N-Methyltrifluormethansulfonylamino-, N-Ethyl-trifluormethansulfonylamino-, N-Isopropyl-trifluormethansulfonylamino-, Methoxycarbonyl-, Ethoxycarbonyl-, Propoxycarbonyl-, Isopropoxycarbonyl-, Methylaminocarbonyl-, Ethylaminocarbonyl-, Dimethylaminocarbonyl, Di-n-propylaminocarbonyl-, N-Methyl-ethylaminocarbonyl-, Trifluormethyl-, Methylaminosulfonyl-, Ethylaminosulfonyl-, n-Propylaminosulfonyl-, n-Butylaminosulfonyl-, n-Pentylaminosulfonyl-, Dimethylaminosulfonyl-, Diethylaminosulfonyl-, Di-n-propylaminosulfonyl-, N-Methyl-isopropylaminosulfonyl-, Acetylamino-, Propionylamino-, Methylcarbonylamino-, Ethylaminocarbonylamino- oder Propylaminocarbonylaminogruppe, eine Methyl-, Ethyl-, Propyl-, Methoxy-, Ethoxy-, Propyloxy-, Allyloxy-, 2-Butenyloxy-, 3-Butenyloxy-, 2-Pentenyloxy-, Propargyloxy-, 2-Butinyloxy-, 3-Butinyloxy-, Cyanmethyloxy-, Cyanethyloxy-, Methoxycarbonylmethyloxy-, Methoxycarbonylethyloxy-, Methylmercapto-, Ethylmercapto-, Methylsulfinyl-, Ethylsulfinyl-, Methylsulfonyl oder Ethylsulfonylgruppe.

Bei Sulfonylgruppen, die durch cyclische Iminogruppen substituiert sein können, sind bevorzugt die Morpholino-, Thiomorpholino-, Pyrrolidino-, Piperidino- und Hexamethyleniminosulfonylgruppen.

Insbesondere sind bevorzugt fur

R₅ Wasserstoff, eine Alkylsulfonyloxy-, Trifluormethylsulfonyloxy-, Alkylsulfenylmethyl-, Alkylsulfinylmethyl-, Alkylsulfonylmethyl-, Alkylsulfonylamino-, N-Alkyl-alkylsulfonylamino-, Trifluormethylsulfonylamino- oder N-Alkyltrifluormethylsulfonylaminogruppe, eine durch eine Hydroxy-, Alkoxy-, Amino-, Alkylamino- oder Dialkylaminogruppe substituierte Carbonylgruppe oder eine durch eine Amino-, Dialkylamino- oder Morpholinogruppe substituierte Sulfonylgruppe, wobei jeder der vorstehend genannten Alkylteile 1 oder 2 Kohlenstoffatome enthalten kann, eine Nitro-, Cyan- oder Alkylaminosulfonylgruppe mit 1-4 Kohlenstoffatomen, eine Alkylcarbonylamino-, Aminocarbonylamino- oder N-Alkyl-aminocarbonylaminogruppe, eine Alkylmercapto-, Alkylsulfinyl- oder Alkylsulfonylgruppe, wobei jeder der vorgenannten Alkylteile 1 oder 2 Kohlenstoffatome enthalten kann, eine Halogen-, Amino-, Hydroxy-, Dialkylamino-, Alkyl-, Alkoxy-, Alkenyloxy- oder Alkinyloxygruppe vorzugsweise mit 1-3 Kohlenstoffatomen, eine Cyanmethyloxy- oder Methoxycarbonylmethyloxygruppe, die Trifluormethylgruppe oder die 1-Imidazolylgruppe,

für R₆ Wasserstoff, eine Alkylgruppe mit 1-3 Kohlenstoffatomen, eine Alkoxy-oder Dialkylaminogruppe mit 1 oder 2 Kohlenstoffatomen in jedem Alkylteil oder ein Halogenatom und

für R₇ Wasserstoff oder die Methoxygruppe.

Der Phenylteil kann 1 bis 3 der genannten Substituenten tragen.

Bevorzugte monosubstituierte Phenylverbindungen sind die Hydroxy-, C₁-C₃ Alkyl-, C₁-C₃ Alkoxy-, Allyloxy-, Propargyloxy-, Cyanmethyloxy-, Methoxycarbonylmethyloxy-, Halogen-, Nitro-, Cyan-, Aminocarbonyl-, Methoxycarbonyl-, Amino-, C₁-C₃ Dialkylamino-, C₁-C₃ Alkylmercapto-, C₁-C₃ Alkylsulfinyl-, C₁-C₃ Alkylsulfonyl-, C₁-C₃ Alkylsulfonyloxy- und die 1-Imidazolyl-phenyle, wobei der Substituent in 2-, 3- oder 4-Stellung stehen kann.

Bevorzugte disubstituierte Phenyle enthalten als Substituenten eine Alkansulfonyloxy-, Trifluormethylsulfonyloxy-, Alkylsulfenylmethyl-, Alkylsulfinylmethyl-, Alkylsulfonylmethyl-, Alkylsulfonylamino-, N-Alkyl-alkylsulfonylamino-, Trifluormethylsulfonylamino- oder N-Alkyl-trifluormethylsulfonylaminogruppe, eine durch eine Hydroxy-, Alkoxy-, Amino-, Alkylamino- oder Dialkylaminogruppe substituierte Carbonylgruppe oder eine durch eine Amino-, Dialkylamino- oder Morpholinogruppe substituierte Sulfonylgruppe, eine Alkylaminosulfonyl-, Alkylcarbonylamino-, Aminocarbonylamino- oder N-Alkyl-aminocarbonylaminogruppe, eine Hydroxy-, Alkyl-, Alkoxy-, Allyloxy-, Propargyloxy-, Cyanmethyloxy-, Methoxycarbonylmethyloxy-, Cyan-, Halogen-, Nitro-, Amino-, Dialkylamino-, Alkylmercapto-, Alkylsulfinyl-, Alkylsulfonyl- oder eine 1-Imidazolylgruppe, wobei die beiden Substituenten gleich oder verschieden sein können und in 2,3-, 2,4-, 2,5-, 2,6-, 3,4- und 3,5-Stellung, bevorzugt jedoch in 2,4-, 2,5- und 3,4-Stellung stehen können und die vorgenannten Alkylreste, allein oder in Kombination mit anderen Resten, 1-3 C-Atomen aufweisen können.

Bevorzugter trisubstituierter Phenylrest ist der 3,4,5-Trimethoxyphenylrest.

Bedeutet R₁ einen heterocyclischen Fünfring mit 1-4 Heteroatomen oder einen heterocyclischen Sechsring mit 1-5 Heteroatomen, wobei die Heteroatome der vorgenannten Fünf- oder Sechsringe gleich oder veschieden sein können und Stickstoff, Sauerstoff oder Schwefel bedeuten und gegebenenfalls an einem oder mehreren Stickstoffatomen ein Sauerstoff tragen können, so sind in diesem Sinne bevorzugt der Pyrrol-, Furan-, Thiophen-, Pyrazol-, Imidazol-, Thiazol-, Isothiazol-, Oxazol-, Isoxazol-, Triazol-, Tetrazol-, Thiadiazol-, Oxadiazol-, Pyrazin-, N,Nʹ-Dioxy-pyrazin-, Pyrimidin-, N,Nʹ-Dioxy-pyrimidin-, Pyridazin-, Oxazin-, Thiazin-, Triazin-, Tetrazin-, Pyridin-, N-Oxy-pyridin-, Piperidin-, Piperazin-, Morpholin- und Thiomorpholinrest.

Alkyl-, Alkoxy- und Alkylmercapto-Substituenten in den heterocyclischen Fünf- und Sechsringen können 1-6, vorzugsweise 1-4 Kohlenstoffatome enthalten. Bevorzugt ist der Methyl-, Ethyl-, Methoxy-, Ethoxy-, Methylmercapto- und Ethylmercaptorest. Unter Halogen ist Fluor, Chlor und Brom, vorzugsweise Chlor zu verstehen.

Sind die heterocyclischen Fünf- und Sechsringe mit einem Phenylring kondensiert, so sind bevorzugt der Indol-, Indazol-, Benzimidazol-, Chinolin-, Isochinolin-, Cinnolin-, Phthalazin-, Chinazolin-, Chinoxalin-, Benzofuran-, Benzothiophen-, Benzoxazol-, Benzisoxazol-, Benzothiazol- und der Benzisothiazolrest, außerdem der Naphthylrest.

Bedeutet X eine Bindung und R₁ einen Alkyl-, Alkenyl- oder Alkinylrest, so sind darunter gerade oder verzweigte Ketten mit bis zu zehn C-Atomen zu verstehen. Bevorzugt in diesem Sinne ist der Methyl-, Ethyl-, Propyl-, Butyl-, Pentyl-, Hexyl-, Vinyl-, Propenyl- und Propinylrest. Bedeutet X eine Bindung und R₁ einen Cycloalkyl- oder Cycloalkenylrest, so sind darunter Ringe mit drei bis sieben Gliedern zu verstehen. Bevorzugt in diesem Sinne ist der Cyclopropyl-, Cyclobutyl-, Cyclopentyl-, Cyclohexyl-, Cyclopentenyl- und Cyclohexenylrest. Bedeutet X eine Bindung und R₁ einen Alkoxyalkyl-, Carboxyalkyl-, Alkoxycarbonylalkyl-, Hydroxyalkyl-, Aminoalkyl-, Alkylamino- oder einen Alkylcarbonylaminorest, so können die Alkyl- oder Alkoxygruppen 1 bis 6 C-Atome enthalten.

Bevorzugt in diesem Sinne ist der Ethoxymethyl-, Methoxyethyl-, Ethoxyethyl-, Carboxymethyl-, Carboxypropyl-, Carboxybutyl-, Methoxycarbonylmethyl-, Methoxycarbonylethyl-, Methoxycarbonylpropyl-, Ethoxycarbonylmethyl-, Ethoxycarbonylethyl-, Ethoxycarbonylpropyl-, Propoxycarbonylethyl-, Aminomethyl-, Aminoethyl-, Aminopropyl-, Aminobutyl-, Hydroxymethyl-, Hydroxyethyl-, Hydroxypropyl-, Hydroxybutyl-, Methylamino-, Ethylamino-, Propylamino-, Butylamino-, Acetylamino-, Propionylamino- oder der Methylsulfonylaminorest.

Unter Halogen ist Fluor und Chlor, vorzugsweise Fluor zu verstehen.

Bedeuten in der allgemeinen Formel I R₂ und R₃ Alkylgruppen, so sind darunter gerade oder verzweigte Alkylketten mit 1-6 C-Atomen zu verstehen. Bevorzugt in diesem Sinne sind die Methyl-, Ethyl-, Propyl- und die Butylgruppe.

Bilden R₂ und R₃ zusammen mit dem C-Atom, an das sie gebunden sind einen carbocyclischen Ring, so sind darunter Ringe mit drei bis sieben Gliedern zu verstehen. Bevorzugt sind der Cyclopropan-, Cyclobutan-, Cyclopentan- und der Cyclohexanring.

Bedeutet in der allgemeinen Formel I R₄ eine Alkylcarbonylamino-, eine Alkylaminocarbonyl-. Dialkylamicoarbonyl- oder Alkylsulfonylaminogruppe, so sind darunter geradkettige, verzweigte und cyclische Alkylgruppen mit 1-6 C-Atomen zu verstehen.

Bevorzugt in diesem Sinne ist die Methylaminocarbonyl-, Ethylaminocarbonyl-, Propylaminocarbonyl-, Butylaminocarbonyl-, Dimethylaminocarbonyl-, Methyl-ethyl-aminocarbonyl-, Diethylaminocarbonyl-, Cyclohexyl-methylaminocarbonyl-, Acetylamino-, Propionylamino-, Butylcarbonylamino-, Methylsulfonylamino-, Ethylsulfonylamino und die Propylsulfonylaminogruppe.

Bedeutet in der allgemeinen Formel I X eine Alkylengruppe, so sind darunter Alkylengruppen mit 1-4 C-Atomen zu verstehen. Bevorzugt in diesem Sinne ist die Methylen- und Ethylengruppe.

Besonders bevorzugte Verbindungen sind Verbindungen der allgemeinen Formel I

in der

R₁ den Phenylrest der allgemeinen Formel II bedeutet, in der
R₅ Wasserstoff, die Methansulfonyloxy-, Trifluormethansulfonyloxy-, Methansulfonylamino-, Trifluormethansulfonylamino-, Methansulfonyl-methylamino-, Trifluormethansulfonyl-methylamino-, Methylsulfenylmethyl-, Methylsulfinylmethyl-, Methylsulfonylmethyl-, Aminocarbonyl-, Aminosulfonyl-, Methylaminosulfonyl-, Dimethylaminosulfonyl-, Acetylamino-, Methylmercapto-, Methylsulfinyl-, Methylsulfonyl-, Hydroxy-, Methyl-, Methoxy-, Propargyloxy-, Cyanmethyloxy-, Methoxycarbonylmethyloxy-, Cyan-, Chlor-, Nitro-, Amino-, Dimethylamino-, Trifluormethyl- oder die 1-Imidazolylgruppe,
R₆ Wasserstoff, die Methyl-, Methoxy-, Dimethylamino- oder Chlorgruppe bedeutet,

R₇ Wasserstoff oder die Methoxygruppe ist

oder

R₁ den Pyrrol-, Furan-, Thiophen-, Pyrazol-, Imidazol-, Isothiazol-, Thiazol-, Oxazol-, Triazol-, Tetrazol-, Thiadiazol-, Isoxazol-, Oxadiazol-, Pyridin-, N-Oxy-pyridin-, Pyrazin-, N,NʹDioxy-pyrazin, Pyrimidin-, N,Nʹ-Dioxypyrimidin-, Pyridazin-, Oxazin-, Thiazin-, Triazin- oder Tetrazinrest darstellt, sowie deren Methyl-, Ethyl-, Methoxy-, Ethoxy-, Methylmercapto-, Ethylmercapto- und chlorsubstituierten Derivate, oder in den Indol-, Indazol-, Chinolin-, Isochinolin-, Pyrrolidin-, Piperidin-, Morpholin-, Thiomorpholin- oder Naphthylrest bedeutet,

oder

R₁ für den Fall, daß X eine Bindung darstellt, neben den genannten Gruppen auch ein Wasserstoffatom, die Methyl-, Ethyl-, Propyl-, Butyl-, Pentyl-, Hexyl-, Propenyl-, Cyclopentenyl-, Cyclohexyl-, Trifluormethyl-, Hydroxy-, Mercapto-, Methylmercapto-, Amino-, Acetylamino-, Formylaminogruppe bedeutet,

R₂ und R₃ gleich sind und Methylgruppen bedeuten, oder
R₂ und R₃ zusammen einen Cyclopentanring bilden,

R₄ die Cyano-, Aminocarbonyl-, Amino-, Formylamino-, Acetylamino-, Isopropionylamino-, tert.-Butylcarbonylamino-, Trifluormethylcarbonylamino-, Methylsulfonylamino-, n-Propylsulfonylamino-, i-Propylsulfonylamino-, Trifluormethylsulfonylamino-, Phenylsulfonylamino-, Methylaminocarbonylamino- oder die Acetylaminocarbonylaminogruppe bedeutet,

X eine Bindung, die Methylengruppe, die Iminogruppe oder eine Carbonylaminogruppe darstellt,

n den Wert 0 oder 1 annimmt.

Die Verbindungen der allgemeinen Formel I können nach an sich bekannten Verfahren hergestellt werden. Besonders vorteilhaft sind die in folgendem Schema dargestellten Synthesewege.

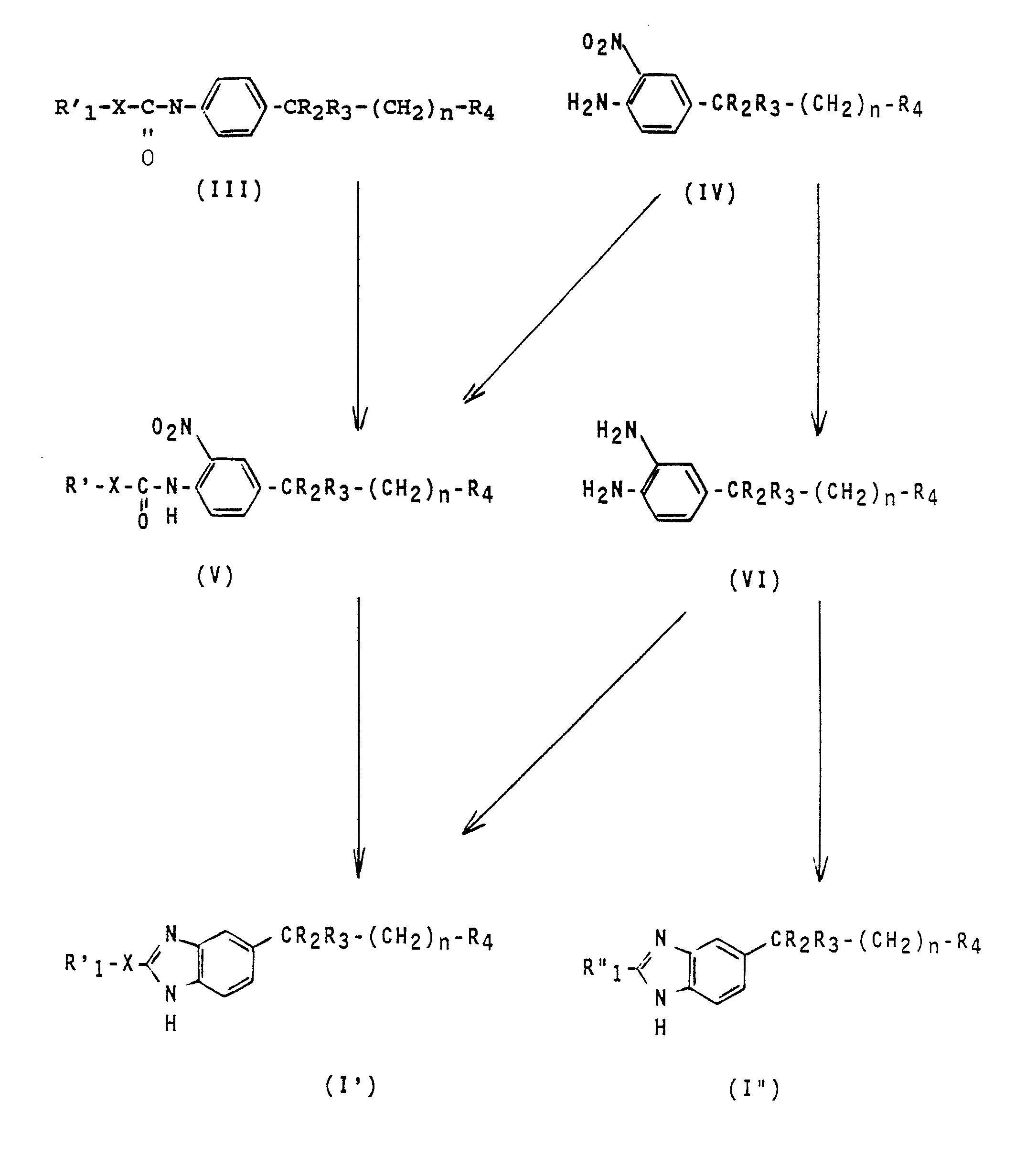

Die Verbindungen der allgemeinen Formel Iʹ, in der X, R₂, R₃, R₄ und n die angegebene Bedeutung besitzen und Rʹ₁ die oben für R₁ angegebenen Bedeutungen besitzt mit der Ausnahme, daß für den Fall, daß X eine Bindung darstellt, Rʹ₁ nicht die Amino-, Hydroxy- oder Mercaptogruppe bedeutet, werden hergestellt, in dem man entweder
a) eine Verbindung der allgemeinen Formel V, in der Rʹ₁, X, R₂, R₃, R₄ und n die angegebenen Bedeutungen besitzen zu den Verbindungen der allgemeinen Formel Iʹ reduziert und cyclisiert, oder
b) eine Verbindung der allgemeinen Formel VI, in der R₂, R₃, R₄ und n die oben angegebenen Bedeutungen besitzt, mit einer Verbindung der allgemeinen Formel VII

   Rʹ₁-X- 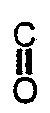 -Y (VII)

   in der Rʹ₁ und X die angegebenen Bedeutungen besitzen und

   Y entweder ein Wasserstoffatom, die Hydroxygruppe oder eine leicht abspaltbare Gruppe bedeutet, zu Verbindungen der allgemeinen Formel Iʹumsetzt.

   Verbindungen der allgemeinen Formel Iʺ, in der Rʺ₁ die Amino-, Hydroxy- oder Mercaptogruppe bedeutet und R₂, R₃, R₄ und n die oben angegebenen Bedeutungen besitzen, stellt man her, indem man
c) Verbindungen der allgemeinen Formel VI, in der R₂, R₃, R₄ und n die angegebenen Bedeutungen besitzen, mit Reagenzien umsetzt, die Carbonyl-, Thiocarbonyl- oder Iminogruppe ubertragen, wie z.B. Phosgen, Thiophosgen, 1,1ʹ-Carbonyldiimidazol, Chlorameisensäureester, Harnstoff oder Bromcyan.

Die nach Verfahren a) - c) erhaltene Verbindung der allgemeinen Formel I oder deren Tautomer kann anschließend gewünschtenfalls in eine andere Verbindung der allgemeinen Formel I umgewandelt und/oder in ein physiologisch verträgliches Salz einer organischen oder anorganischen Säure überführt werden.
Die in Verfahren a) genannte Reduktion wird vorzugsweise in einem Lösungsmittel oder Lösungsmittelgemisch wie Wasser, Methanol, Ethanol, Eisessig, Essigsäureethylester oder Dimethylformamid mit Wasserstoff in Gegenwart eines Katalysators wie Raney Nickel, Platin oder Palladium/Kohle, mit Metallen wie Eisen, Zinn oder Zink in Gegenwart einer Säure, mit Salzen wie Eisen(II)sulfat, Zinn(II)chlorid, Natriumsulfid, Natriumhydrogensulfit oder Natriumdithionit oder mit Hydrazin in Gegenwart von Raney-Nickel bei Temperatur zwischen 0 und 100°C, vorzugsweise jedoch bei Raumtemperatur, durchgefuhrt. Dabei entstehen meist unmittelbar die cyclisierten Verbindungen der allgemeinen Formel Iʹ.

Die Cyclisierung kann gewünschtenfalls vervollständigt werden, indem man nach der Reduktion vorzugsweise in einem Lösungsmittel oder Lösungsmittelgemisch wie Ethanol, Isopropanol, Eisessig, Benzol, Toluol, Chlorbenzol, Glycol, Ethylenglycoldimethylether, Sulfolan oder Dimethylformamid auf Temperaturen zwischen 50 und 220°C, vorzugsweise jedoch auf die Siedetemperatur des Reaktionsgemisches, gegebenenfalls in Gegenwart eines Kondensationsmittels wie Phosphoroxychlorid, Thionylchlorid, p-Toluolsulfonsäure, Salzsäure, Schwefelsäure, Phosphorsäure, Polyphosphorsäure oder gegebenenfalls auch in Gegenwart einer Base wie Natriumhydroxid, Natriummethylat oder Kalium-tert.-butylat erhitzt. Die Cyclisierung kann jedoch auch ohne Lösungsmittel und/oder Kondensationsmittel durchgefuhrt werden.

Unter den in Verfahren b) genannten Verbindungen der allgemeinen Formel VII versteht man Aldehyde, Carbonsäuren, Säurehalogenide wie Säurechloride, Carbonsäureester wie Methyl- und Ethylester und andere aktivierte Carbonsäurederivate sowie Anhydride.

Ist die Verbindung der allgemeinen Formel VII ein Aldehyd, so findet die Umsetzung mit Verbindungen der allgemeinen Formel VI unter oxidierenden Bedingungen statt, vorzugsweise in alkoholischem Medium unter Erwärmen zum Rückfluß in Gegenwart von Luftsauerstoff und katalytischen Mengen Säure, wie Toluolsulfonsäure, oder in Gegenwart von Luftsauerstoff und eines Katalysators wie Braunstein in saurem Milieu wie z.B. Eisessig bei Raumtemperatur. Vielfach ist es von Vorteil, den Aldehyd zuerst durch Umsetzung mit NaHSO₃ in das Bisulfit-Addukt zu überführen, welches unter den eben für den Aldehyd beschriebenen Bedingungen weiter umgesetzt wird.

Ist die Verbindung der allgemeinen Formel VII eine Carbonsäure so findet die Umsetzung mit Verbindungen der allgemeinen Formel VI in Gegenwart eines wasserentziehenden Mittels, vorzugsweise in Polyphosphorsäure bei Temperaturen zwischen 50 und 250°C, vorzugsweise zwischen 100 und 200°C statt.

Ist die Verbindung der allgemeinen Formel VII ein Carbonsäurederivat, so findet die Umsetzung mit Verbindungen der allgemeinen Formel VI in einem inerten Lösungsmittel, vorzugsweise in Methylenchlorid oder Pyridin statt. Zur Vervollständigung der Cyclisierung erhitzt man anschließend in einem Lösungsmittel oder Lösungsmittelgemisch wie Ethanol, Isopropanol, Eisessig, Benzol, Chlorbenzol, Glycol, Diethylenglycoldimethylether, Sulfolan oder Dimethylformamid auf Temperaturen zwischen 50 und 250°C, vorzugsweise jedoch auf die Siedetemperatur des Lösungsmittels oder Lösungsmittelgemisches, gegebenenfalls in Gegenwart eines Kondensationsmittels wie Phosphoroxychlorid, Thionylchlorid, p-Toluolsulfonsäure, Salzsäure, Schwefelsäure, Phosphorsäure, Polyphosphorsäure oder gegebenenfalls auch in Gegenwart einer Base wie Natriumhydroxid, Kaliummethylat oder Kalium-tert.-butylat. Die Cyclisierung kann jedoch auch ohne Lösungsmittel und/oder Kondensationsmittel durchgeführt werden.

Die in Verfahren c) beschriebenen Cyclisierungen der Verbindungen der allgemeinen Formel V zu Verbindungen der allgemeinen Formel IIʺ fuhrt man vorzugsweise so aus, daß man in eine salzsaure Lösung der Verbindungen der allgemeinen Formel VI Phosgen einleitet oder Thiophosgen zugibt und bei Raumtemperatur stehen läßt, oder die Verbindungen der allgemeinen Formel VI mit Bromcyan oder Harnstoff ohne Lösungsmittel erhitzt, oder die Verbindungen der allgemeinen Formel VI mit 1,1ʹ-Carbonyldiimidazol in einem inerten Lösungsmittel wie Dioxan zum Sieden erhitzt.

Die als Ausgangsmaterial benötigten Verbindungen der allgemeinen Formel V, in der R₁, R₂, R₃, R₄, X und n die angegebenen Bedeutungen besitzen, stellt man her
a) durch Nitrierung von Verbindungen der allgemeinen Formel III oder
b) durch Umsetzung von Verbindungen der allgemeinen Formel IV mit Carbonsäuren oder Carbonsäurenderivaten der allgemeinen Formel VIII

   R₁ʹ-X- 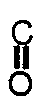 -Z (VIII)

   in der R₁ʹ und X die angegebenen Bedeutungen besitzen und Z die Hydroxygruppe oder einen leicht abspaltbaren Rest darstellt.

Die als Ausgangsmaterial benötigten Verbindungen der allgemeinen Formel VI, in der R₂, R₃, R₄ und n die angegebenen Bedeutungen besitzen, werden durch Reduktion von Verbindungen der allgemeinen Formel IV erhalten.

Die Verbindungen der allgemeinen Formel III und IV sind literaturbekannt oder können nach literaturbekannten Verfahren erhalten werden.

Die Umwandlungen von Verbindungen der allgemeinen Formel I zu anderen Verbindungen der allgemeinen Formel I trifft zum Beispiel zu
a) für die Umsetzung einer Verbindung der allgemeinen Formel I, in der R₁ eine Amino- oder Aminoalkylgruppe, oder einen gesättigten Stickstoffheterocyclus darstellt, oder einen mit einer Aminogruppe substituierten heterocyclischen Fünf- oder Sechsring darstellt, oder einen Phenylring der allgemeinen Formel II darstellt, in der ein oder mehrere der Substituenten R₅, R₆, R₇ eine Aminogruppe darstellen, und/oder in der R₄ eine Aminogruppe darstellt, mit Carbonsäuren oder mit aktivierten Carbonsäurederivaten wie Anhydriden oder Säurehalogeniden zu Formylamino- oder Alkylcarbonylaminoderivaten. Umsetzungen mit Carbonsäuren führt man vorzugsweise in Gegenwart eines wasserentziehenden Mittels wie z.B. Polyphosphorsäure oder eines mit Wasser ein azeotropes Gemisch bildenden Lösungsmittel wie Benzol oder Toluol durch. Umsetzungen mit aktivierten Carbonsäurederivaten werden vorzugsweise in inerten Lösungsmitteln wie Methylenchlorid oder Pyridin bei Temperaturen zwischen 0 und 250°C, vorzugsweise jedoch bei der Siedetemperatur des Lösungsmittels durchgeführt.
b) fur die Umsetzung von Verbindungen der allgemeinen Formel I, in der R₁ eine Amino- oder Aminoalkylgruppe, oder einen gesättigten Stickstoffheterocyclus darstellt, oder einen mit einer Aminogruppe substituierten heterocyclischen Fünf- oder Sechsring darstellt, wie er eingangs definiert ist, oder R₁ einen Phenylring der allgemeinen Formel II darstellt, in welcher einer der Substituenten R₅, R₆, R₇ eine Amino-, N-Alkylamino- oder Hydroxygruppe darstellt, und/oder R₄ eine Aminogruppe darstellt, mit einer Sulfonsäure der allgemeinen Formel IX

   R₈-SO₂OH (IX),

   in der R₈ eine Alkylgruppe mit 1-3 Kohlenstoffatomen oder eine Trifluormethylgruppe darstellt oder mit einem reaktionsfähigen Derivat hiervon, zu Verbindungen der allgemeinen Formel I, in welcher die be-sagten Amino-, Aminoalkyl-, cyclischen Imino-, N-Alkylamino- oder Hydroxygruppen sulfoniert sind.
   Die Umsetzung wird zweckmäßigerweise in einem Lösungsmittel oder Lösungsmittelgemisch wie Methylenchlorid, Ether, Tetrahydrofuran, Dioxan oder Benzol gegebenenfalls in Gegenwart eines säurebindenden Mittels wie Natriumcarbonat, Triethylamin oder Pyridin, wobei die beiden letzteren gleichzeitig auch als Lösungsmittel verwendet werden können, in Gegenwart eines die Säure aktivierenden oder wasserentziehenden Mittels wie Thionylchlorid oder Phosphorpentachlorid, vorzugsweise jedoch mit einem reaktionsfähigen Derivat einer Verbindung der allgemeinen Formel IX, z.B. mit deren Anhydrid oder Halogenid wie Methansulfonsäurechlorid oder Ethansulfonsäurechlorid, vorzugsweise bei Temperaturen zwischen 0 und 100°C, z.B. bei Temperaturen zwischen Raumtemperatur und 50°C, durchgeführt.
c) fur die Umwandlung von Verbindungen der allgemeinen Formel I, in der R₁ eine Phenylgruppe der allgemeinen Formel II darstellt, wobei einer der Substituenten R₅, R₆, R₇ eine Alkylmercapto- oder Alkylsulfenylmethylgruppe mit 1-3 Kohlenstoffatomen im Alkylteil ist, zu Verbindungen der allgemeinen Formel I, in der R₁ eine Phenylgruppe und einer der Substituenten R₅, R₆, R₇ eine Alkylsulfinyl-, Alkylsulfonyl-, Alkylsulfinylmethyl- oder Alkylsulfonylmethylgruppe darstellt.
   Diese Oxidation wird vorzugsweise in einem Lösungsmittel oder Lösungsmittelgemisch, z.B. Wasser, Wasser/Pyridin, Aceton, Eisessig, verdünnter Schwefelsäure oder Trifluoressigsäure, je nach dem verwendeten Oxidationsmittel zweckmäßigerweise bei Temperaturen zwischen -80 und 100°C durchgeführt.
   Zur Herstellung einer Alkylsulfinyl- oder Alkylsulfinylmethylverbindung der allgemeinen Formel I wird die Oxidation zweckmäßigerweise mit einem Aequivalent des verwendeten Oxidationsmittel durchgeführt, z.B. mit Wasserstoffperoxid in Eisessig, Trifluoressigsäure oder Ameisensäure bei 0 bis 20°C oder in Aceton bei 0 bis 60°C, mit einer Persäure wie Perameisensäure in Eisessig oder Trifluoressigsäure bei 0 bis 50°C oder mit m-Chlorperbenzoesäure in Methylenchlorid oder Chloroform bei -20°C bis 60°C, mit Natriummetaperjodat in wässrigem Methanol oder Ethanol bei -15 bis 25°C, mit Brom in Eisessig oder wässriger Essigsäure, mit N-Brom-succinimid in Ethanol, mit tert.-Butylhypochlorid in Methanol bei -80°C bis -30°C, mit Jodbenzodichlorid in wässrigem Pyridin bei 0 bis 50°C, mit Salpetersäure in Eisessig bei 0 bis 20°C, mit Chromsäure in Eisessig oder in Aceton bei 0 bis 20°C und mit Sulfurylchlorid in Methylenchlorid bei -79°C, der hierbei erhaltene Thioether-Chlor-Komplex wird zweckmäßigerweise mit wässrigem Ethanol hydrolisiert.
   Zur Herstellung einer Alkylsulfonyl- oder Alkylsulfonylmethylverbindung der allgemeinen Formel I wird die Oxidation zweckmäßigerweise mit einem bzw. mit zwei oder mehr Aequivalenten des verwendeten Oxidationsmittels durchgeführt, z.B. Wasserstoffperoxid in Eisessig, Trifluoressigsäure oder in Ameisensäure bei 20 bis 100°C oder in Aceton bei 0 bis 60°C, mit einer Persäure wie Perameisensäure, m-Chlorperbenzoesäure in Eisessig, Trifluoressigsäure, Methylenchlorid oder Chloroform bei Temperaturen zwischen 0 und 65°C, mit Salpetersäure in Eisessig bei 0 bis 20°C, mit Chromsäure oder Kaliumpermanganat in Eisessig, Wasser/Schwefelsäure oder in Aceton bei 0 bis 20°C.
d) fur die Umwandlung von Verbindungen der allgemeinen Formel I, in der R₁ eine Phenylgruppe der allgemeinen Formel I darstellt, wobei einer der Substituenten R₅, R₆, R₇ eine Carboxyl- oder Hydroxysulfonylgruppe darstellt, zu Verbindungen der allgemeinen Formel I, in der einer der Substituenten R₅, R₆ und R₇ eine durch eine Amino-, Alkylamino- oder Dialkylaminogruppe substituierte Carbonyl- oder Sulfonylgruppe darstellt. Dies geschieht durch Umsetzung mit einem Amin HNR₉R₁₀, wobei R₉ und R₁₀ gleich oder verschieden sein können und Wasserstoff oder C₁-C₅ Alkylgruppen bedeuten, oder mit einem reaktionsfähigen Derivat hiervon. Vorteilhaft ist es, die Carboxylgruppe oder Hydroxysulfonylgruppe in ein reaktionsfähiges Derivat, z.B. in einen Ester oder ein Säurechlorid zu verwandeln und dann mit dem Amin HNR₉R₁₀ umzusetzen.
   Die Umsetzung wird zweckmäßigerweise in einem Lösungsmittel oder Lösungsmittelgemisch wie Methylenchlorid, Ethanol, Chloroform, Tetrachlorkohlenstoff, Ether, Tetrahydrofuran, Dioxan, Benzol, Toluol, Acetonitril oder Dimethylformamid, gegebenenfalls in Gegenwart eines die Säure aktivierenden Mittels oder eines wasserentziehenden Mittels, z.B. in Gegenwart von Chlorameisensäureethylester, Thionylchlorid, Phosphortrichlorid, Phosphorpentoxid, N,N-Dicyclohexylcarbodiimid/N-Hydroxysuccinimid, N,Nʹ-Carbonyldiimidazol oder N,Nʹ-Thionyldiimidazol oder Triphenylphosphin/Tetrachlorkohlenstoff, oder eines die Aminogruppe aktivierenden Mittels, z.B. Phosphortrichlorid, und gegebenenfalls in Gegenwart einer anorganischen Base wie Natriumcarbonat oder einer tertiaeren organischen Base wie Triethylamin oder Pyridin, welche gleichzeitig als Lösungsmittel dienen können, bei Temperaturen zwischen -25 und 250°C, vorzugsweise jedoch bei Temperaturen zwischen -10°C und der Siedetemperatur des verwendeten Lösungsmittels durchgeführt werden, desweiteren kann während der Umsetzung entstehendes Wasser durch azeotrope Destillation, z.B. durch Erhitzen mit Toluol am Wasserabscheider, oder durch Zugabe eines Trockenmittels wie Magnesiumsulfat oder Molekularsieb abgetrennt werden.
   Besonders vorteilhaft wird jedoch die Umsetzung in einem entsprechenden Halogenid, z.B. dem Carbonsäure- oder Sulfonsäurechlorid, und einem entsprechenden Amin, wobei diese gleichzeitig als Lösungsmittel dienen können, und bei Temperaturen zwischen 0 und 50°C durchgeführt.
   Die oben erwähnten Reaktionsbedingungen treffen auch zu auf die Herstellung von Verbindungen der allgemeinen Formel I, in der R₄ eine Alkylaminocarbonyl- oder Dialkylaminocarbonylgruppe bedeutet, aus Verbindungen der allgemeinen Formel I, in der R₄ die Carboxylgruppe oder ein reaktionsfähiges Derivat hiervon bedeutet.
e) für die Umwandlung von Verbindungen der allgemeinen Formel I, in der R₁ eine Phenylgruppe der allgemeinen Formel II darstellt, wobei einer der Substituenten R₅, R₆, R₇ die Cyangruppe und/oder in der R₄ die Cyangruppe darstellt zu Verbindungen der allgemeinen Formel I, in welcher R₁ einen Phenylring der allgemeinen Formel II darstellt und einer der Substituenten R₅, R₆ oder R₇ eine Alkoxycarbonylgruppe, eine Aminocarbonylgruppe oder eine Carboxylgruppe bedeutet und/oder in der R₄ eine Aminocarbonylgruppe darstellt.
   Diese Alkoholyse und/oder Hydrolyse wird entweder in Gegenwart einer Säure wie Salzsäure, Schwefelsäure, Phosphorsäure oder Trichloressigsäure oder in Gegenwart einer base wie Natriumhydroxid oder Kaliumhydroxid in einem geeigneten Lösungsmittel wie Wasser, Wasser/Methanol, Ethanol, Wasser/Ethanol, Wasser/Isopropanol oder Wasser/Dioxan bei Temperaturen zwischen -10 und 120°C, z.B. bei Temperaturen zwischen Raumtemperatur und der Siedetemperatur des Reaktionsgemisches, durchgeführt.
f) für die Umwandlung von Verbindungen der allgemeinen Formel I, in der R₄ die Cyano- oder Aminocarbonylgruppe bedeutet zu solchen Verbindungen der allgemeinen Formel I, in der R₄ eine Aminogruppe bedeutet. Bei dieser Reduktion erhöht sich der Wert von n auf n + 1.
   Diese Reduktion führt man in inerten Lösungsmitteln mittels Lithiumalanat, Natriumborhydrid in Gegenwart von Kobaltchlorid, mit Boran oder durch katalytische Hydrierung bei erhöhten Temperaturen und Drücken aus.
g) für die Alkylierung von Verbindungen der allgemeinen Formel I, in welcher R₁ einen Phenylring der allgemeinen Formel II darstellt, in der einer der Substituenten R₅, R₆ oder R₇ eine Hydroxy- oder Mercaptogruppe bedeutet, oder in welcher R₁ einen mit einer Hydroxy- oder Mercaptogruppe substituierten heterocyclischen Ring darstellt oder in der X eine Bindung darstellt und R₁ eine Hydroxy- oder Mercaptogruppe bedeutet. Dabei entstehen die entsprechenden Alkylmercapto- oder Alkoxyverbindungen.
   Die Reaktionen werden vorzugsweise in einem Lösungsmittel wie Aceton, Ether, Benzol, Toluol oder Dimethylformamid bei Temperaturen zwischen -30°C und +100°C, vorzugsweise bei Raumtemperatur in Gegenwart einer Base wie Kaliumcarbonat oder Natriumhydrid und eines Alkylierungsmittels wie Alkylhalogeniden oder Alkylsulfaten durchgeführt.
h) für die Reduktion von Verbindungen der allgemeinen Formel I, in welcher R₁ einen Pyridinring darstellt, zu Verbindungen der allgemeinen Formel I, in der R₁ den Piperidinring bedeutet. Diese Reduktionen führt man vorzugsweise in alkoholischen Medium in Gegenwart eines Katalysators wie Platin oder Palladium mittels Wasserstoff bei Normaldruck oder leicht erhöhtem Druck und einer Temperatur zwischen Raumtemperatur und 60°C.
i) für die Hydrierung einer Vinylverbindung (X = -CH=CH-) in eine entsprechende Ethylverbindung (X = -CH₂-CH₂-). Die Hydrierung wird vorzugsweise in einem Lösungsmittel wie Wasser, Wasser/Ethanol, Methanol, Eisessig, Essigsäureethylester oder Dimethylformamid zweckmäßigerweise mit Wasserstoff in Gegenwart eines Hydrierungskatalysators wie Raney-Nickel, Platin oder Palladium/Kohle durchgeführt.
k) für die Oxidation eines Fünf- oder Sechsringes mit einem oder mehreren Stickstoffatomen zu den entsprechenden N-Oxiden. Die Oxidation wird zweckmäßig mit einem oder mehreren Aequivalenten des verwendeten Oxidationsmittels durchgeführt, z.B. mit Wasserstoffperoxid in Eisessig, Trifluoressigsäure oder in Ameisensäure bei 20 - 100°C oder in Aceton bei 0 - 60°C, mit einer Persäure wie Perameisensäure oder m-Chlorperbenzoesäure in Eisessig, Trifluoressigsäure, Methylenchlorid oder Chloroform bei Temperaturen zwischen 0 und 60°C.
l) für die Hydrierung von Verbindungen der allgemeinen Formel I, in der R₁ einen ungesättigten Heterocyclus bedeutet, zu Verbindungen der allgemeinen Formel I, in der R₁ einen gesättigten Heterocyclus darstellt. Diese Reduktion führt man bevorzugt in einem inerten Lösungsmittel wie Wasser, Methanol, Ethanol, Eisessig, Essigsäureethylester oder Dimethylformamid mit Wasserstoff in Gegenwart eines Hydrierungskatalysators wie Raney-Nickel, Platin oder Palladium durch.
m) für die Umsetzung von Verbindungen der allgemeinen Formel I, in der R₄ eine Aminogruppe darstellt, zu Verbindungen der allgemeinen Formel I, in der R₄ eine Dialkylaminocarbonylaminogruppe darstellt. Diese Reaktion führt man bevorzugt mit Dialkylcarbamidsäurechloriden in einem inerten Lösungsmittel wie Dichlormethan in Gegenwart einer Base wie Triethylamin bei 0-50°C durch.

Ferner können die so erhaltenen Verbindungen der allgemeinen Formel I anschließend gewünschtenfalls in ihre physiologisch verträglichen säureadditionssalze mit anorganischen oder organischen säuren überführt werden. Als Säuren kommen hierfür beispielsweise Salzsäure, Bromwasserstoffsäure, Schwefelsäure, Phosphorsäure, Fumarsäure, Bernsteinsäure, Weinsäure, Zitronensäure, Milchsäure, Maleinsäure oder Methansulfonsäure in Betracht.

Wie bereits eingangs erwähnt weisen die neuen Verbindungen der allgemeinen Formel I, deren Tautomere und deren physiologisch verträgliche säureadditionssalze bei einer langen Wirkungsdauer überlegene pharmakologische Eigenschaften auf, insbesondere eine blutdrucksenkende und/oder positiv inotrope Wirkung und/oder beeinflussen die Thrombozytenfunktion und verbessern die Mikrozirkulation.

Zur Herstellung von Arzneimitteln werden die Substanzen der allgemeinen Formel I in an sich bekannter Weise mit geeigneten pharmazeutischen Trägersubstanzen, Aroma-, Geschmacks- und Farbstoffen gemischt und beispielsweise als Tabletten oder Dragees ausgeformt oder unter Zugabe entsprechender Hilfsstoffe in Wasser oder Öl, wie z.B. Olivenöl, suspendiert oder gelöst.

Die erfindungsgemäßen neuen Substanzen der allgemeinen Formel I und ihre Salze können in flüssiger oder fester Form enteral oder parenteral appliziert werden. Als Injektionsmedium kommt vorzugsweise Wasser zur Anwendung, welches die bei InjektionsLösungen üblichen Zusätze wie Stabilisierungsmittel, Lösungsvermittler oder Puffer enthält.

Derartige Zusätze sind z.B. Tartrat- und Citratpuffer, Ethanol, Komplexbildner (wie Ethylendiamintetraessigsäure und deren nicht toxische Salze) und hochmolekulare Polymere (wie flussiges Polyethylenoxid) zur Viskositätsregulierung. Feste Trägerstoffe sind z.B. Stärke, Lactose, Mannit, Methylcellulose, Talkum, hochdisperse Kieselsäuren, hochmolekulare Fettsäuren (wie Staerinsäure), Gelatine, Agar-Agar, Calciumphosphat, Magnesiumstearat, tierische und pflanzliche Fette und feste hochmolekulare Polymere (wie Polyethylenglykole). Für orale Applikation geeignete Zubereitungen können gewünschtenfalls Geschmacks- und Sußstoffe enthalten.

Die erfindungsgemaeßen Verbindungen werden ueblicherweise in Mengen von 10-500 mg pro Tag bezogen auf 75 kg Koerpergewicht appliziert. Bevorzugt ist es, 2-3 mal pro Tag 1-2 Tabletten mit einem Wirkstoffgehalt von 5-200 mg zu verabreichen. Die Tabletten koennen auch retardiert sein, wodurch nur noch 1mal pro Tag 1-2 Tabletten mit 10-500 mg Wirkstoff gegeben werden muß. Der Wirkstoff kann auch durch Injektion 1-8mal pro Tag bzw. durch Dauerinfusion gegeben werden, wobei Mengen von 5-200 mg/Tag normalerweise ausreichen.

Besonders bevorzugt im Sinne der vorliegenden Erfindung sind außer den in den Beispielen genannten Verbindungen die folgenden und deren Tautomere:

5-(2-Formylaminomethyl-propan-2-yl)-2-(4-pyridazinyl)benzimidazol
5-(2-Formylaminomethyl-propan-2-yl)-2-(2-pyrazinyl)benzimidazol
5-(2-Formylaminomethyl-propan-2-yl)-2-(2-hydroxypyridin-4-yl)benzimidazol
5-(2-Formylaminomethyl-propan-2-yl)-2-(3-hydroxypyridin-4-yl)benzimidazol
5-(2-Formylaminomethyl-propan-2-yl)-2-(2,6-dihydroxypyridin-4-yl)benzimidazol
5-(2-Formylaminomethyl-propan-2-yl) 2-(2-chlorpyridin-4-yl)benzimidazol
5-(2-Formylaminomethyl-propan-2-yl)-2-(2-methyl-pyridin-5-yl)benzimidazol
5-(2-Formylaminomethyl-propan-2-yl)-2-(5-n-butylpyridin-2-yl)benzimidazol
5-(2-Formylaminomethyl-propan-2-yl)-2-(6-hydroxypyridazin-3-yl)benzimidazol

5-(2-Formylaminomethyl-propan-2-yl)-2-[2-(3-pyridinyl)ethenyl]benzimidazol
5-(2-Formylaminomethyl-propan-2-yl)-2-[2-(3-pyridinyl)ethyl]benzimidazol
5-(2-Formylaminomethyl-propan-2-yl)-2-(2-pyrrolyl)benzimidazol
5-(2-Formylaminomethyl-propan-2-yl)-2-(3-thienyl)benzimidazol
5-(2-Formylaminomethyl-propan-2-yl)-2-thenylbenzimidazol
5-(2-Formylaminomethyl-propan-2-yl)-2-(4-thiazolyl)benzimidazol
5-(2-Formylaminomethyl-propan-2-yl)-2-(5-isoxazolyl)benzimidazol
5-(2-Formylaminomethyl-propan-2-yl)-2-(2-methyloxazol-4-yl)benzimidazol
5-(2-Formylaminomethyl-propan-2-yl)-2-(5-methylpyrazol-3-yl)benzimidazol
5-(2-Formylaminomethyl-propan-2-yl)-2-(1,2,4-triazol-3-yl)benzimidazol
5-(2-Formylaminomethyl-propan-2-yl)-2-[(5-carboxy)-1,2,3-triazol-4-yl]benzimidazol
5-(2-Formylaminomethyl-propan-2-yl)-2-[(5-methoxycarbonyl)-1,2,3-triazol-4-yl]benzimidazol
5-(2-Formylaminomethyl-propan-2-yl)-2-(1,2,3-thiadiazol-4-yl)benzimidazol
5-(2-Formylaminomethyl-propan-2-yl)-2-(1,2,3-thiadiazol-5-yl)benzimidazol
5-(2-Formylaminomethyl-propan-2-yl)-2-(1,3,4-thiadiazol-2-yl)benzimidazol
5-(2-Formylaminomethyl-propan-2-yl)-2-[(5-methylmercapto)-1,3,4-oxadiazol-2-yl]benzimidazol
5-(2-Formylaminomethyl-propan-2-yl)-2-(3-chinolinyl)benzimidazol
5-(2-Formylaminomethyl-propan-2-yl)-2-(2-indolyl)benzimidazol
5-(2-Formylaminomethyl-propan-2-yl)-2-(1-naphthylmethyl)benzimidazol
5-(2-Formylaminomethyl-propan-2-yl)-2-phenylbenzimidazol
5-(2-Formylaminomethyl-propan-2-yl)-2-(2-methoxyphenyl)benzimidazol
5-(2-Formylaminomethyl-propan-2-yl)-2-(2-methoxy-4-methylsulfinyl-phenyl)benzimidazol
5-(2-Formylaminomethyl-propan-2-yl)-2-(2-methoxy-4-methylsulfonyl-phenyl)benzimidazol
5-(2-Formylaminomethyl-propan-2-yl)-2-[4-(1-imidazolyl)phenyl]benzimidazol
5-(2-Formylaminomethyl-propan-2-yl)-2-(4-Chlorphenyl)benzimidazol
5-(2-Formylaminomethyl-propan-2-yl)-2-(4-trifluormethylphenyl)benzimidazol
5-(2-Formylaminomethyl-propan-2-yl)-2-(2-acetamidophenyl)benzimidazol
5-(2-Formylaminomethyl-propan-2-yl)-2-(4-tert.-butylphenyl)benzimidazol
5-(2-Formylaminomethyl-propan-2-yl)-2-n-propylbenzimidazol
5-(2-Formylaminomethyl-propan-2-yl)-2-i-propylbenzimidazol
5-(2-Formylaminomethyl-propan-2-yl)-2-i-propenylbenzimidazol
5-(2-Formylaminomethyl-propan-2-yl)-2-tert.-butyl-benzimidazol
5-(2-Formylaminomethyl-propan-2-yl)-2-cyclopropyl-benzimidazol
5-(2-Formylaminomethyl-propan-2-yl)-2-(1-cyclopentenyl)benzimidazol
5-(2-Formylaminomethyl-propan-2-yl)-2-(2-carboxy-ethyl)benzimidazol
5-(2-Formylaminomethyl-propan-2-yl)-2-methoxymethyl-benzimidazol
5-(2-Formylaminomethyl-propan-2-yl)-2-(3-aminopropyl)benzimidazol
5-(2-Formylaminomethyl-propan-2-yl)-2-mercapto-benzimidazol
5-(2-Formylaminomethyl-propan-2-yl)-2-methylmercapto-benzimidazol
5-(2-Formylaminomethyl-propan-2-yl)-2-amino-benzimidazol
5-(2-Formylaminomethyl-propan-2-yl)-2-(4-pyridinylamino)benzimidazol
5-(2-Formylaminomethyl-propan-2-yl)-2-(2-thiazolylamino)benzimidazol
5-(2-Formylamino-ethyl)-2-(4-pyridinyl)benzimidazol
5-(1-Formylamino-propan-2-yl)-2-(4-pyridinyl)benzimidazol

5-(1-Formylamino-butan-2-yl)-2-(4-pyridinyl)benzimidazol
5-(1-Formylamino-2-methyl-butan-2-yl)-2-(4-pyridinyl)benzimidazol

5-[2-(tert.-Butylcarbonylaminomethyl)-propan-2-yl)-2-(4-methoxyphenyl)benzimidazol
5-[2-(Trifluormethylcarbonylaminomethyl)-propan-2-yl)-2-(4-pyridinyl)benzimidazol
5-[2-(Methylsulfonylaminomethyl)-propan-2-yl]-2-(2-methoxy-4-methylsulfonyl-phenyl)benzimidazol
5-[2-(n-Propylsulfonylaminomethyl)-propan-2-yl]-2-(4-methoxyphenyl)benzimidazol
5-[2-(i-Propylsulfonylaminomethyl)-propan-2-yl]-2-(4-methoxyphenyl)benzimidazol
5-[2-(Trifluormethylsulfonylaminomethyl)-propan-2-yl]-2-(4-methoxyphenyl)benzimidazol
5-[2-(Phenylsulfonylaminomethyl)-propan-2-yl]-2-(4-methoxyphenyl)benzimidazol
5-[2-(Methylaminocarbonylaminomethyl)-propan-2-yl]-2-(4-pyridinyl)benzimidazol
5-[2-(Acetylaminocarbonylaminomethyl)-propan-2-yl]-2-(4-pyridinyl)benzimidazol
5-(2-Methyl-2-formylamino-propan-2-yl)-2-(4-methoxyphenyl)benzimidazol
5-(3-Formylaminopropyl)-2-(4-methoxyphenyl)benzimidazol
5-(2-Formylaminopropyl)-2-(4-pyridinyl)benzimidazol
5-(2-Methyl-2-formylamino-propan-2-yl)-2-(4-pyridinyl)benzimidazol
5-(3-Formylaminopropyl)-2-(4-pyridinyl)benzimidazol
5-(2-Methyl-3-formylamino-propyl)-2-(4-pyridinyl)benzimidazol
5-(1-Methyl-4-methylaminocarbonyl-butyl)-2-(4-pyridinyl)benzimidazol
5-(1-Methylaminocarbonyl-propan-2-yl)-2-(4-pyridinyl)benzimidazol
5-(1-Aminocarbonyl-propan-2-yl)-2-(4-pyridinyl)benzimidazol
5-[1-(N-Cyclohexyl-N-methyl-aminocarbonyl)-propan-2-yl]-2-(4-pyridinyl)benzimidazol
5-(2-Cyan-propan-2-yl)-2-(2-methoxy-2-methylmercaptophenyl)benzimidazol
5-(2-Cyan-propan-2-yl)-2-(2-methoxy-4-methylsulfonylphenyl)benzimidazol
5-(2-Cyan-propan-2-yl)-2-(2-methoxy-4-methylsulfonylphenyl)benzimidazol
5-(2-Aminocarbonyl-propan-2-yl)-2-(4-methoxy-phenyl)benzimidazol
5-[2-[(3,3-Dimethyl-oxindol-5-yl)-methylaminomethyl]propan-2-yl]-2-(4-pyridinyl)benzimidazol
5-(1-Methyl-2-methylaminocarbonyl-ethyl)-2-(4-pyridinyl)benzimidazol
5-(2-Formylaminomethyl-propan-2-yl)-2-(5-pyrimidinyl)benzimidazol

### Beispiel 1

### 5-(2-Aminomethyl-propan-2-yl)-2-(4-pyridinyl)benzimidazol

a) 63.7 g 4-(2-Cyano-propan-2-yl)anilin in 1 l Toluol versetzte man mit 50 ml Essigsäureanhydrid. Nach 30 min kühlte man auf Raumtemperatur ab, rührte mit Natriumbicarbonatlösung durch, trennte die organische Phase ab und engte im Vak. zur Trockene ein. Den Rückstand nahm man in Toluol auf, saugte die Kristalle ab und trocknete an der Luft. Man erhielt 73.6 g (91 %) 4-(2-Cyano-propan-2-yl)acetanilid mit dem Schmp. 113-114°C.
b) 84.3 g 4-(2-Cyano-propan-2-yl)acetanilid hydrierte man in 400 ml Ethanol in Gegenwart von 300 ml flüssigem Ammoniak und 5 g Raney-Nickel bei 90°C und 50 bar Wasserstoffdruck sechs Stunden bei 90°C. Nach Absaugen des Katalysators und Eindampfen des Filtrate s erhielt man nach Kristallisation aus Toluol 72.5 g (84 %) 4-(2-Aminomethyl-propan-2-yl)acetanilid als farblose Kristalle mit dem Schmp. 97-101°C.
c) Zu 72.5 g 4-(2-Aminomethyl-propan-2-yl)acetanilid in 400 ml Dichlormethan tropft man unter Eiskühlung 36.5 ml Essigsäureanhydrid. Anschließend rührte man eine Stunde bei Raumtemperatur, engte im Vak. ein, digerierte mit Essigester, saugte ab und wusch mit Essigester. Man erhielt 82.0 g (94 %) N,Nʹ-Diacetyl-4-(2-aminomethyl-propan-2-yl)anilin als farblose Kristalle mit dem Schmp. 171-172°C.
d) Zu 125 g N,Nʹ-Diacetyl-4-(2-aminomethyl-propan-2-yl)anilin in 315 ml konz. Schwefelsäure tropfte man bei 0°C eine Mischung aus 39 ml 65 % Salpetersäure (d = 1.4) und 59 ml konz. Schwefelsäure. Man rührte noch 30 min bei Raumtemperatur, goß auf Eis und extrahierte mit Dichlormethan. Die organische Phase schüttelte man mit wässriger Natriumbicarbonatlösung und engte die organische Phase im Vak. ein. Den zähen Rückstand nahm man in 200 ml Essigester auf, setzte bis zur beginnenden Trübung Ether zu und ließ kristallisieren. Man saugte ab, wusch mit Essigester/Ether (2:1) und erhielt 122 g (83 %) N,Nʹ-Diacetyl-4-(2-aminomethyl-propan-2-yl)-2-nitroanilin als schwach gelbe Kristalle mit dem Schmp. 138-139°C.
e) Zu 25 g Kaliumhydroxid in 1 l Methanol gab man 122 g N,Nʹ-Diacetyl-4-(2-aminomethyl-propan-2-yl)-2-nitroanilin und rührte 1 Stunde bei Raumtemperatur. Man neutralisierte mit Essigester, engte im Vak. zur Trockene ein, digerierte mit Wasser, saugte die Kristalle ab, wusch mit Wasser nach und trocknete an der Luft. Man erhielt 101 g (97 %) 4-(2-Acetamidomethyl-propan-2-yl)-2-nitro-anilin als gelbe Kristalle mit dem Schmp. 132-134°C.
f) 12.5 g 4-(2-Acetamidomethyl-propan-2-yl)-2-nitroanilin hydrierte man in Gegenwart von 1 g 10 % Palladium auf Kohle bei Normaldruck und Raumtemperatur. Nachdem 3.6 1 Wasserstoff aufgenommen waren, saugte man den Katalysator ab und engte das Filtrat im Vak. ein. Man erhielt 11 g (100 %) 4-(2-Acetamidomethyl-prop-2-yl)-1,2-diaminobenzol als bräunliches Öl, welches man ohne weitere Reinigung einsetzte.
g) Zu 22.1 g 4-(2-Acetamidomethyl-propan-2-yl)-1,2-diamino-benzol und 42 ml Triethylamin in 500 ml Dichlormethan gab man unter Kühlung mit Eiswasser 27 g Isonicotinsäurechlorid-Hydrochlorid. Man rührte bei Raumtemperatur bis zur vollständigen Umsetzung des Diamins (Dünnschichtchromatographie, Kieselgel, Dichlormethan: methanol. Ammoniak = 10:1). Man schüttelte mit Wasser, trennte die organische Phase ab und engte im Vak. zur Trockene ein. Den Rückstand (24.3 g) nahm man in 200 ml Ethanol auf, versetzte mit 60 ml konz. Salzsäure und erhitzte drei Tage unter Rückfluß zum Sieden. Man engte im Vak. zur Trockene ein, digerierte den Ruckstand mit wässriger Ammoniaklosung bei pH = 6, gab 50 ml Dichlormethan zu, saugte ab und wusch mit Eiswasser und Dichlormethan nach. Man erhielt 14.1 g farblose Kristalle, die man säulenchromatographisch reinigte (Säule: Durchmesser 6 cm. Länge 40 cm, gefüllt mit 1 l Kieselgel, Laufmittel Dichlormethan: methanol. Ammoniak = 15:1). Nach Vereinigung entsprechender Fraktionen, Entfernung des Laufmittels im Vak. und Anreiben mit Essigester erhielt man 9.2 g der Titelverbindung als farblose Kristalle mit dem Schmp. 199-202°C. 2.7 g davon kristallisierte man aus Dioxan um und erhielt 2.3 g farblose Kristalle mit dem Schmp. 203-204°C.

### Beispiel 2

### 5-(2-Formylaminomethyl-propan-2-yl)-2-(4-pyridinyl)benzimidazol

9.1 g 5-(2-Aminomethyl-propan-2-yl)-2-(4-pyridinyl)benzimidazol (Beispiel 1) löste man in 10 ml Ameisensäure und engte im Vak. ein. Den Rückstand erhitzte man 2 Stunden mit 100 ml Toluol am Wasserabscheider zum Sieden. Man engte im Vak. zur Trockene ein, nahm den Rückstand in einer Mischung aus Dichlormethan und Methanol (5:2) auf, versetzte mit wässrigem Ammoniak und rieb bis zur Kristallisation an. Man saugte ab und wusch mit Wasser und Dichlormethan nach. Man erhielt 9.4 g farblose Kristalle, die man aus Wasser/Ethanol (2:1) umkristallisierte. Man erhielt 8.7 g (86 %) der Titelverbindung, der pro Mol noch 1/2 Mol Wasser anhaftete. Die Verbindung schmilzt bei 115-121°C unter Abgabe des Kristallwassers, kristallisiert erneut, um dann bei 227-228°C erneut zu schmelzen.

### Beispiel 3

Durch Umsetzung des in Beispiels 1 f dargestellten 4-(2-Acetamidomethyl-propan-2-yl)-1,2-diaminobenzols mit den genannten säurechloriden nach der Vorschrift in Beispiel 1 g erhielt man die folgenden Verbindungen:
a) durch Umsetzung mit 2-Pyridincarbonylchlorid erhielt man 5-(2-Aminomethyl-propan-2-yl)-2-(2-pyridinyl)benzimidazol in 87 % Ausbeute als Öl.
b) durch Umsetzung mit 4-Pyridylessigsäurechlorid erhielt man 5-(2-Aminomethyl-propan-2-yl)-2-(4-pyridinyl-methyl)benzimidazol in 91 % Ausbeute.
c) durch Umsetzung mit 4-Chinolincarbonylchlorid erhielt man 5-(2-Aminomethyl-propan-2-yl)-2-(4-chinolinyl)-benzimidazol in 72 % Ausbeute.
d) durch Umsetzung mit 6-Chlornicotinsäurechlorid erhielt man 5-(2-Aminomethyl-propan-2-yl)-2-(6-hydroxy-pyridin-3-yl)benzimidazol in 84 % Ausbeute.
e) durch Umsetzung mit 2-Chlor-6-methylnicotinsäurechlorid erhielt man 5-(2-Aminomethyl-propan-2-yl)-2-(2-hydroxy-6-methyl-pyridin-3-yl)benzimidazol in 35 % Ausbeute.
f) durch Umsetzung mit 3,4-Dimethoxybenzoesäurechlorid erhielt man 5-(2-Aminomethyl-propan-2-yl)-2-(3,4-dimethoxyphenyl)benzimidazol in 11 % Ausbeute.
g) durch Umsetzung mit 2-Methoxy-4-chlorbenzoesäurechlorid erhielt man 5-(2-Aminomethyl-propan-2-yl)-2-(2-methoxy-4-chlorphenyl)benzoesäure in 75 % Ausbeute.

### Beispiel 4

Die in Beispiel 3 erhaltenen Amine formylierte man analog der Vorschrift in Beispiel 2 und erhielt
a) aus 10.2 g des in Beispiel 3 a hergestellten Amins nach Versetzen mit 10 g N-Cyclohexylsulfamidsäure in Essigester in 60 % Ausbeute 5-(2-Formylaminomethyl-propan-2-yl)-2-(2-pyridinyl)benzimidazol als Cyclaminat mit einem Schmp. 170°C (Zers.) nach Kristallisation aus Wasser.
b) aus 3.2 g des in Beispiel 3 b hergestellten Amins 1.9 g 5-(2-Formylaminomethyl-propan-2-yl)-2-(4-pyridinylmethyl)benzimidazol als beige Kristalle mit dem Schmp. 141-148°C, die man zur Reinigung in wassergesättigtem Essigester löste, von geringen unlöslichen Bestandteilen absaugte und am Wasserabscheider zum Sieden erhitzte. Nach Abkühlen erhielt man so 1.2 g (34 %) beige Kristalle mit dem Schmp. 153-156°C.
c) aus 14.5 des in Beispiel 3 c hergestellten Amins 10.0 g 5-(2-Formylaminomethyl-propan-2-yl)-2-(4-chinolinyl)benzimidazol als farblose Kristalle mit dem Schmp. 183-185°C, welches man zur Reinigung aus der Hülse durch wenig Kieselgel mit 200 ml Aceton extrahierte. Man erhielt so 7.6 g (48 %) farblose Kristalle mit dem Schmp. 186-189°C.
d) aus 10.5 g des in Beispiel 3 d hergestellten Amins 5.6 g (49 %) 5-(2-Formylaminomethyl-propan-2-yl)-2-(6-hydroxy-pyridin-3-yl)benzimidazol nach zweimaligem Umkristallisieren aus Wasser/Ethanol als farblose Kristalle mit dem Schmp. 211-215°C.
e) aus 4.4 g des in Beispiel 3 e hergestellten Amins 4.5 g 5-(2-Formylaminomethyl-propan-2-yl)-2-(2-hydroxy-6-methyl-pyridin-3-yl)benzimidazol als als Öl.
f) aus 0.6 g des in Beispiel 3 f hergestellten Amins 0.5 g 5-(2-Formylaminomethyl-propan-2-yl)-2-(3,4-dimethyoxyphenyl)benzimidazol nach säulenchromatographischer Trennung (Kieselgel, CH₂Cl₂:CH₃OH = 80:20) mit dem Schmp. 287-288°C.
g) aus 4.9 g des in Beispiel 3 g hergestellten Amins 1.0 g 5-(2-Formylaminomethyl-propan-2-yl)-2-(2-methoxy-4-chlorphenyl)benzimidazol mit dem Schmp. 156-157°C nach Umkristallisation aus Essigester.
h) aus 1.5 g des in Beispiel 43 hergestellten Amins 0.5 g 5-(2-Formylaminomethyl-propan-2-yl)-2-(2-furyl)benzimidazol mit dem Schmp. 130-135°C.
i) aus 1.9 g des in Beispiel 44 hergestellten Amins 0.7 g 5-(2-Formylaminomethyl-propan-2-yl)-2-(thienyl)benzimidazol mit dem Schmp. 157-159°C.
j) aus 2.6 g des in Beispiel 34 hergestellten 5-(2-Aminomethyl-propan-2-yl)-2-(2-methoxy-4-methylmercapto-phenyl)benzimidazol 2.4 g 5-(2-Formylaminomethyl-propan-2-yl)-2-(2-methoxy-4-methylmercapto-phenyl)benzimidazol nach Kristallisation aus Ethanol/H₂O = 4:1 mit dem Schmp. 122-124°C als Hydrat.
k) aus 3.0 g des in Beispiel 41 hergestellten Amins 2.2 g 5-(2-Formylaminomethyl-propan-2-yl)-2-phenyl-benzimidazol als harten Schaum mit dem Schmelzintervall 98-110°C.
l) aus 3.3 g des in Beispiel 42 hergestellten Amins 0.6 g 5-(2-Formylaminomethyl-propan-2-yl)-2-(4-hydroxyphenyl)benzimidazol als Öl.

Analog den Beispielen 4 stellte man folgende Verbindungen her:

5-(2-Formylaminomethyl-propan-2-yl)-2-(1,2,5-thiadiazol-3-yl)benzimidazol
5-(2-Formylaminomethyl-propan-2-yl)-2-[(3,3-dimethyl-2-oxindol-5-yl)methyl]benzimidazol
5-(2-Formylaminomethyl-propan-2-yl) 2-(4-dimethylaminophenyl)benzimidazol
5-(2-Formylaminomethyl-propan-2-yl)-2-(4-hydroxy-3,5-dimethxy-phenyl)benzimidazol
5-(2-Formylaminomethyl-propan-2-yl)-2-(2-phenyl-ethenyl)benzimidazol
5-(2-Formylaminomethyl-propan-2-yl)-2-benzimidazol
5-(2-Formylaminomethyl-propan-2-yl)-2-(1-propan-1-yl)benzimidazol
5-(2-Formylaminomethyl-propan-2-yl)-2-hexylbenzimidazol
5-(2-Formylaminomethyl-propan-2-yl)-2-cyclohexylbenzimidazol
5-(2-Formylaminomethyl-propan-2-yl)-2-(2-ethoxycarbonylethyl)benzimidazol
5-(2-Formylaminomethyl-propan-2-yl)-2-hydroxybenzimidazol
5-(2-Formylaminomethyl-propan-2-yl)-2-(4-methoxyphenylamino)benzimidazol
5-(2-Formylamino-propan-2-yl)-2-(4-pyridinyl)benzimidazol
5-(2-Formylamino-2-methyl-propan-2-yl)-2-(4-methoxyphenyl)benzimidazol
5-(2-Formylaminopropyl-2-(4-methoxyphenyl)benzimidazol
5-(2-Formylaminomethyl-propan-2-yl)-2-(4-methoxyphenyl)benzimidazol
5-(2-Formylaminomethyl-propan-2-yl)-2-phenylbenzimidazol
5-(2-Formylaminomethyl-propan-2-yl)-2-(4-hydroxyphenyl)benzimidazol

### Beispiel 5

### 5-(2-Aminomethyl-propan-2-yl)-2-(3-pyridinyl)benzimidazol

a) 6.3 g 4-(2-Acetamido-propan-2-yl)-2-nitro-anilin, dessen Herstellung in Beispiel 1 e beschrieben ist, legte man in 50 ml trockenem Pyridin vor, kühlte mit Eiswasser und trug portionsweise 4.9 g Nicotinsäurechlorid-Hydrochlorid ein, wobei die Temperatur unterhalb +15°C gehalten wurde. Nach 2 Stunden Rühren bei Raumtemperatur verdünnte man mit Eiswasser auf das dreifache Volumen, saugte die Kristalle ab und wusch mit Wasser. Man erhielt 8.2 g (96 %) 4-(2-Acetamidomethyl-propan-2-yl)-2-nitro-1-nicotinoylaminobenzol als schwach gelbe Kristalle mit dem Schmp. 194-196°C.
b) 8.2 g 4-(2-Acetamidomethyl-propan-2-yl)-2-nitro-1-nicotinoylamino-benzol hydrierte man in 100 ml Methanol in Gegenwart von 0.8 g 10 % Palladium auf Kohle bei Normaldruck und Raumtemperatur. Nachdem 1.5 l Wasserstoff aufgenommen waren, filtrierte man und engte das Filtrat im Vak. ein. Den Rückstand (7.8 g) versetzte man mit 100 ml Ethanol und 30 ml konz. Salzsäure und erhitzte drei Tage unter Rückfluß zum Sieden. Man engte im Vak. ein, nahm den Rückstand in wenig Wasser auf und stellte mit Natronlauge alkalisch. Man extrahierte mit einer Mischung Dichlormethan:Methanol = 10:1, trocknete die organische Phase, filtrierte und engte im Vak. ein. Man erhielt 4.4 g (69 %) der Titelverbindung als spröde Masse.

### Beispiel 6

### 4-(2-Aminomethyl-propan-2-yl)-2-(4-methoxyphenyl)benzimidazol

Durch Umsetzung von 10 0 g 4-(2-Acetamidomethyl-propan-2-yl)-2-nitro-anilin, dessen Darstellung in Beispiel 1 e beschrieben ist, mit 7.5 g Anissäurechlorid analog der Vorschrift in Beispiel 5 a erhielt man 14.3 g (93 % 4-(2-Acetamidomethyl-propan-2-yl)-2-nitro-1-(4-methoxyphenylcarbonylamino)benzol mit dem Schmp. 124-126°C als gelbe Kristalle. Daraus erhielt man analog der Vorschrift in Beispiel 5 b die Titelverbindung in 98 % Ausbeute mit einem Schmp. von 328-331°C als Hydrochlorid.

### Beispiel 7

Die in Beispiel 5 und 6 erhaltenen Amine formylierte man analog dem Beispiel 2 und erhielt
a) aus 4.4 g des in Beispiel 5 hergestellten Amins 2.2 g (45 %) 5-(2-Formylaminomethyl-propan-2-yl)-2-(3-pyridinyl)benzimidazol als harten Schaum mit einem Schmelzintervall von 60-120°C.
b) aus 11.8 g des Hydrochlorids des in Beispiel 6 hergestellten Amins 9.8 g (5-(2-Formylaminomethyl-propan-2-yl)-2-(4-methoxyphenyl)benzimidazol als farblose Kristalle mit dem Schmp. 165-170°C, die man zur Reinigung in heißem Aceton löste und Essigester bis zur beginnenden Trübung zugab. Man erhielt 6.1 g (47 %) farblose Kristalle mit dem Schmp. 167-169°C.

### Beispiel 8

### 5-(2-Aminomethyl-propan-2-yl)-2-benzyl-benzimidazol

a) 115 g 4-(2-Cyano-propan-2-yl)-1-nitro-benzol in 600 ml Ethanol hydrierte man in Gegenwart von 500 ml flüssigem Ammoniak und 5 Löffeln Raney-Nickel bei einem Druck von 120 bar Wasserstoff und einer Temperatur von 90°C sechs Stunden. Man saugte ab, engte im Vak. ein und erhielt 96 g 4-(2-Aminomethyl-propan-2-yl)anilin als Öl.
b) Zu 109 ml Essigsäureanhydrid tropfte man 55 ml Ameisensäure, erwärmte 15 min auf 50°C und kühlte auf Raumtemperatur ab. Diese Lösung tropfte man zu 72.3 g 4-(2-Aminomethyl-propan-2-yl)anilin in 1 l Dichlormethan unter Rühren und Eiskühlung. Man rührte 30 min bei Raumtemperatur, versetzte mit Natriumcarbonatlösung, trennte die organische Phase ab und engte im Vak. ein. Den Rückstand nahm man in Dichlormethan auf, saugte die Kristalle ab und wusch nach. Man erhielt 43.7 g (45 %) N,N,-Diformyl-4-(aminomethyl-propan-2-yl)anilin mit dem Schmp. 138-140°C als farblose Kristalle.
c) In 195 ml 96 % Salpetersäure (d = 1.5) trug man unter Rühren und Kühlen portionsweise 43.0 g N,Nʹ-Diformyl-4-(aminomethyl-propan-2-yl)anilin so ein, daß die Temperatur bei 0°C gehalten wird. Man rührte noch 30 min bei dieser Temperatur, goß auf 1.5 kg Eis, neutralisierte sofort durch Zugabe von 400 Kaliumcarbonat in 2 l Wasser und extrahierte mit einer Mischung aus Dichlormethan:Methanol = 20:1. Die organsiche Phase trocknete man mit Magnesiumsulfat, filtrierte und engte im Vak. ein. Den Rückstand reinigte man säulenchromatographisch (2 l Kieselgel, Essigester als Laufmittel). Man erhielt nach Vereinigung entsprechender Fraktionen und Entfernung des Laufmittels im Vak. 9.3 g (18 %) N,Nʹ-Diformyl-4-(2-aminomethyl propan-2-yl)-2-nitro-anilin als gelbe Kristalle mit dem Schmp. 90-95°C.
d) 40 ml Methanol sättigte man bei 20°C mit Ammoniak und löste darin 9.3 g N,Nʹ-Diformyl-4-(2-aminomethyl-propan-2-yl)-2-nitro-anilin. Nach zwanzig Stunden engte man im Vak. ein, nahm den Rückstand in Dichlormethan auf und schüttelte zweimal mit Wasser aus. Man trocknete die organische Phase, filtrierte und engte im Vak. ein. Man erhielt 9.2 g gelbe Kristalle. Eine Probe davon kristallisierte man aus Toluol/Essigester 2 l um und erhielt 4-(2-Formylamino-methyl-propan-2-yl)-2-nitro-anilin mit dem Schmp. 113-115°C.
e) Zu 4.6 g 4-(2-Formylaminomethyl-propan-2-yl)-2-nitro-anilin in 20 ml absolutem Pyridin tropfte man unter Kühlen mit Eiswasser 3.3 g Phenacetylchlorid. Man rührte eine Stunde bei Raumtemperatur, gab Dichlormethan und Eis zu, säuerte mit Salzsäure bis pH = 1 an, trennte die organische Phase ab und entfernte das Lösungsmittel im Vak.. Den Rückstand reinigte man säulenchromatographisch (Durchmesser 6 cm, Länge 40 cm, gefüllt mit 800 ml Kieselgel, Laufmittel: Essigester) Entsprechende Fraktionen wurden vereinigt und das Laufmittel im Vak. entfernt. Den Ruckstand (5 3 g, 77 %) hydrierte man in 1000 ml Methanol in Gegenwart von 0.5 g 10 % Palladium auf Kohle bei Normaldruck und Raumtemperatur. Nachdem 950 ml Wasserstoff aufgenommen waren, saugte man ab und engte das Filtrat im Vak. ein. Den Rückstand (4.6 g) erhitzte man in 100 ml Ethanol und 10 ml konz. Salzsäure eine Stunde unter Rückfluß zum Sieden. Man engte im Vak. ein, stellte den Rückstand mit wässriger Ammoniaklösung alkalisch und extrahierte mit Dichlormethan. Man trocknete die organische Phase, filtrierte und engte im Vak. ein. Man erhielt 4.1 g der Titelverbindung (42 %).

### Beispiel 9

Aus 4.2 g 4-(2-Formylaminomethyl-propan-2-yl)-2-nitroanilin, dargestellt in Beispiel 8 d, erhielt man durch Umsetzung mit 2-Methoxy-5-methylmercapto-benzoesäurechlorid (4.2 g) analog dem Beispiel 8 e 4.9 g (66 %) 4-(2-Formylaminomethyl-propan-2-yl)-2-nitro-1-(2-methoxy-5-methylmercapto-benzoylamino)benzol als gelbliche Kristalle mit dem Schmp. 151-157°C. Durch weitere Umsetzung analog dem Beispiel 8 e erhielt man daraus 4.0 g (98 %) 5-(2-Aminomethyl-propan-2-yl)-2-(2-methoxy-5-methylmercapto-phenyl)benzimidazol als Öl.

### Beispiel 10

Die in Beispiel 8 und 9 erhaltenen Amine formylierte man analog dem Beispiel 2 und erhielt
a) aus 4.1 g des in Beispiel 8 erhaltenen Amins 2.7 g 5-(2-Formylaminomethyl-propan-2-yl)-2-benzyl-benzimidazol mit dem Schmp. 120-127°C, welches man aus Aceton mit Bleicherdebehandlung umkristallisierte und so 1.7 g (38 %) farblose Kristalle mit Schmp. 128-130°C erhielt.
b) aus 4.4 g des in Beispiel 9 erhaltenen Amins 4.7 g 5-(2-Formylaminomethyl-propan-2-yl)-2-(2-methoxy-5-methylmercapto-phenyl)benzimidazol mit dem Schmp. 182-185°C.

### Beispiel 11

### 5-(1-Aminomethyl-cyclopentan-1-yl)-2-(4-pyridinyl)benzimidazol

a) Analog dem Beispiel 8 a erhielt man aus 23 g 4-(1-Cyano-cyclopentan-1-yl)-1-nitro-benzol 18.7 g (93 %) 4-(1-Aminomethyl-cyclopentyl)anilin als gelbliches Öl.
b) Daraus erhielt man analog dem Beispiel 1 c 20.3 g (75 %) N,Nʹ-Diacetyl-4-(1-aminomethyl-cyclopentan-1-yl)anilin als farblose Kristalle mit dem Schmp. 152-153°C.
c) Daraus erhielt man analog dem Beispiel 1 d 17.5 g (74 %) N,Nʹ-Diacetyl-4-(1-aminomethyl-cyclopentan-1-yl)-2-nitro-anilin als gelbliche Kristalle mit dem Schmp. 154-156°C.
d) Daraus erhielt man analog dem Beispiel 1 e 14.2 g (93 %) 4-(1-Acetylaminomethyl-cyclopentan-1-yl)-2-nitro-anilin als gelbe Kristalle mit dem Schmp. 129-133°C.
e) Aus 7.2 g dieser Substanz und 6.1 g Isonicotinoylchlorid-Hydrochlorid erhielt man analog dem Beispiel 8 e 8.2 g (83 %) 4-(1-Acetylaminomethylcyclopentan-1-yl)-2-nitro-1-isonicotinoylamino-benzol mit dem Schmp. 158-160°C und durch weitere Umsetzung analog Beispiel 8 e 5.0 g (81 %) der Titelverbindung als gelbliches Öl.

### Beispiel 12

Das in Beispiel 11 erhaltene Amin formylierte man analog dem Beispiel 2 und erhielt 5.0 g 5-(1-Formylaminomethylcyclopentan-1-yl)-2-(4-pyridinyl)benzimidazol mit dem Schmp. 214-217°C, welches man aus Dioxan:Wasser = 2:1 umkristallisierte und 2.9 g (54 %) gelbe Kristalle mit dem Schmp. 215-217°C erhielt.

### Beispiel 13

### 5-(1-Cyano-cyclopentan-1-yl-2-(4-pyridinyl)benzimidazol

a) 45.0 g 4-(1-Cyano-cyclopentan-1-yl) 1-nitro-benzol hydrierte man in 700 ml Methanol in Gegenwart von 2 g 10 % Palladium auf Aktivkohle. Man filtrierte, entfernte das Lösungsmittel im Vak. und erhielt 36.3 g (94 %) 4-(1-Cyano-cyclopentan-1-yl)anilin als gelbliche Kristalle mit dem Schmp. 83-86°C.
b) 36.3 g 4-(1-Cyano-cyclopentan-1-yl)anilin in 400 ml Toluol versetzte man mit 21 ml Essigsäureanhydrid. Nach 40 min kühlte man auf Raumtemperatur ab, rührte mit Natriumbicarbonatlösung durch, trennte die organische Phase ab, trocknete mit Natriumsulfat, filtrierte und engte das Filtrat im Vak. ein. Den Rückstand digerierte man mit wenig kaltem Toluol, saugte ab und erhielt 35.3 g (79 %) 4-(1-Cyano-cyclopentan-1-yl)acetanilid mit dem Schmp. 111-113°C.
c) Zu einer klaren Lösung von 35.3 g 4-(1-Cyano-cyclopentan-1-yl)-acetanilid in 95 ml konz. Schwefelsäure tropfte man bei 0°C eine Mischung aus 7 ml 65 % Salpetersäure (d = 1.4) und 17 ml konz. Schwefelsäure. Man rührte nach 30 min bei Raumtemperatur, goß die Lösung auf Eis, extrahierte mit Dichlormethan, saugte ungelöste Bestandteile ab, extrahierte die organische Phase mit Natriumbicarbonatlösung, trocknete die organische Phase mit Natriumsulfat, filtrierte und entfernte das Lösungsmittel im Vak.. Den Rückstand reinigte man säulenchromatographisch (Länge 50 cm, Durchmesser 6.5 cm; Kieselgel, Laufmittel: Dichlormethan) und erhielt 10.5 g (29 %) 4-(1-Cyano-cyclopentan-1-yl)-2-nitro-anilin als gelbe Kristalle mit dem Schmp. 125-128°C.
d) 10.5 g 4-(1-Cyano-cyclopentyl)-2-nitro-anilin hydrierte man in 180 ml Methanol in Gegenwart von 0.5 g 10 % Palladium auf Aktivkohle bei Normaldruck und Raumtemperatur. Nachdem 3.1 1 Wasserstoff aufgenommen waren saugte man ab und engte das Filtrat im Vak. ein. Man erhielt 9.1 g (100 %) 4-(1-Cyano-cyclopentan-1-yl)-1,2-diamino-benzol als gelbes Öl.
e) Analog der Vorschrift in Beispiel 1 g setzte man 4.5 g 4-(1-Cyano-cyclopentan-1-yl)-1,2-diamino-benzol mit 6.0 g Isonicotinsäurechlorid-Hydrochlorid um und erheilt 5.7 g der Titelverbindung als HCl-Salz, welches man zweimal aus Ethanol umkristallisierte. Die so gereinigte Verbindung (4.5 g, 58 %) mit dem Schmp. 209-210°C (Zers.) enthält pro Mol Base 1.5 Mol HCl.

### Beispiel 14

Analog der Vorschrift in Beispiel 1 g setzte man 4.5 g des in Beispiel 13 d dargestellten 4-(1-Cyano-cyclopentan-1-yl)-1,2-diamino-benzol mit 5.7 g Anissäurechlorid um und erhielt 4.8 g 5-(1-Cyano-cyclopentan-1-yl)-2-(4-methoxyphenyl)benzimidazol als Hydrochlorid mit dem Schmp. 250-255°C (Zers.), welches man zur Reinigung aus Ethanol umkristallisierte, wodurch man 3.9 g (49 %) farblose Kristalle mit dem Schmp. 253-255°C (Zers.) erhielt.

### Beispiel 15

### 5-(2-Cyano-propan-2-yl)-2-(4-pyridinyl)benzimidazol

a) 90.2 g 4-(2-Cyano-propan-2-yl)-1-nitro-benzol hydrierte man in 1 l Methanol bei 30°C in Gegenwart von drei Löffeln Raney-Nickel bis 33 l Wasserstoff aufgenommen waren. Man filtrierte und engte das Filtrat im Vak. ein. Man erhielt 84.8 g 4-(2-Cyano-propan-2-yl)anilin als gelbliches Öl, das man ohne weitere Reinigung einsetzte.
b) Analog der Vorschrift in Beispiel 13 b erhielt man aus 84.8 g des in a) erhaltenen als 84.3 g 4-(2-Cyano-propan-2-yl)acetanilid als beige Kristalle mit dem Schmp. 105-108°C.
c) 53.1 g 4-(2-Cyano-propan-2-yl)acetanilid in 160 ml konz. Schwefelsäure nitrierte man mit einer Mischung aus 18.2 ml 65 % Salpetersaure (d = 1.4) und 29.5 ml konz. Schwefelsäure und erhielt 38 g 4-(2-Cyano-propan-2-yl)-2-nitro-acetanilid (58 %) nach Umkristallisation aus Essigester als gelbliche Kristalle mit dem Schmp. 110-111°C.
d) 38.0 g 4-(2-Cyano-propan-2-yl)-2-nitro-acetanilid erhitzte man in 200 ml Methanol und 20 ml konz. Salzsäure 1.5 Stunden unter Rückfluß zum Sieden. Man entfernte das Lösungsmittel im Vak., digerierte den Rückstand mit Wasser und erhielt 29.4 g (93 %) 4-(2-Cyano-propan-2-yl)-2-nitro-anilin als orangegelbe Kristalle mit dem Schmp. 97-99°C.
e) Aus 18.5 g 4-(2-Cyano-propan-2-yl)-2-nitro-anilin erhielt man analog der Vorschrift in Beispiel 13 d 15.8 g (100 %) 4-(2-Cyano-propan-2-yl)-1,2-diaminobenzol als bräunliches Öl.
f) Analog der Vorschrift in Beispiel 1 g setzte man 15.9 8 g 4-(2-Cyano-propan-2-yl)-1,2-diaminobenzol mit 24 g Isonicotinsäurechlorid-Hydrochlorid um und erhielt 18.9 g der Titelverbindung, welche man aus Essigester umkristallisierte und so 7.4 g (31 %) schwach rosa gefärbte Kristalle mit dem Schmp. 254-255°C erhielt.

### Beispiel 16

Analog der Vorschrift in Beispiel 1 g setzte man 4.1 g des in 15 e dargestellten 4-(2-Cyano-prop-2-yl)-1,2-diaminobenzols mit 6.0 g Anissäurechlorid um und erhielt 3.9 g 5-(2-Cyano-propan-2-yl)-2-(4-methoxyphenyl)benzimidazol als Hydrochlorid, welches man aus Ethanol umkristallisierte und so 3.2 g (42 %) schwach bläulich gefärbte Kristalle mit dem Schmp. 253-255°C erhielt.

### Beispiel 17

### 5-(2-Isobutyramidomethyl-propan-2-yl)-2-(4-pyridinyl)benzimidazol

3.5 g des in Beispiel 1 hergestellten 5-(2-Aminomethyl-propan-2-yl)-2-(4-pyridinyl)benzimidazols in 30 ml Dichlormethan versetzte man mit 3 ml Isobuttersäureanhydrid. Dabei erwärmte sich der Ansatz und die Substanz ging in Lösung. Man rührte zwei Stunden, gab dann Natriumbicarbonatlösung zu und rührte weiter bis die Substanz zuerst schmierig und dann kristallin ausfiel. Man saugte ab, wusch mit Wasser und erhielt 4.3 g farblose Kristalle mit dem Schmp. 208-211°C, die man in 150 ml wassergesättigtem Essigester löste, auf etwa 100 ml einengte und so 3.4 g (82 %) der Titelverbindung mit dem Schmp. 213-214°C.

### Beispiel 18

Analog dem Beispiel 17 erhielt man durch Umsetzung mit Acetanhydrid in 85 % Ausbeute 5-(2-Acetamidomethyl-propan-2-yl)-2-(4-pyridinyl)benzimidazol als farblose Kristalle nach Umkristallisation aus Essigester mit dem Schmp. 219-225°C.

### Beispiel 19

### 5-(2-Methylsulfonylaminomethyl-propan-2-yl)-2-(4-pyridinyl)benzimidazol

Zu 2.0 g des in Beispiel 1 hergestellten 5-(2-Aminomethyl-propan-2-yl)-2-(4-pyridinyl)benzimidazols und 830 mg Triethylamin in 50 ml trockenem Dichlormethan tropfte man unter Kühlen mit Eiswasser 953 mg Mesylchlorid, rührte 10 min und gab dann 20 ml Wasser zu. man saugte ab, wusch mit Wasser und Dichlormethan, kristallisierte aus Wasser/Ethanol = 2:1 um und erhielt 2.0 g (85 %) der Titelverbindung als Hydrat in Form gelblicher Kristalle, die ab 130°C sintern, ab 150°C erneut kristallisieren und dann bei 215-216°C schmelzen.

### Beispiel 20

### 5-(2-Formylaminomethyl-propan-2-yl)-2-(4-piperidinyl)benzimidazol

3.0 g des in Beispiel 2 hergestellten 5-(2-Formylaminomethyl-propan-2-yl)-2-(4-pyridinyl)benzimidazols in 50 ml Methanol hydrierte man in Gegenwart von 1 ml Eisessig und 1 g 10 % Palladium auf Kohle sechs Stunden bei 50°C und 5 bar Wasserstoffdruck. Man filtrierte, engte das Filtrat im Vak. ein, digerierte den Rückstand mit Essigester, saugte ab, löste den Rückstand in 40 ml heißem Dimethylformamid und filtrierte. Das Filtrat versetzte man nach dem Abkühlen mit der gleichen Menge Essigester. Nach Kristallisation saugte man ab, wusch mit Essigester und erhielt 1.8 g (59 %) der Titelverbindung als farblose Kristalle mit dem Schmp. 234-237°C.

### Beispiel 21

### 5-(2-Formylaminomethyl-propan-2-yl)-2-(2-methoxy-5-methylsulfonyl-phenyl)benzimidazol

Zu 4.7 g des in Beispiel 10 b hergestellten 5-(2-Formylaminomethyl-propan-2-yl)-2-(2-methoxy-5-methylmercapto-phenyl)benzimidazols in 130 ml 80 % Ameisensäure gab man 10 ml Wasserstoffperoxid und erwärmte zwei Stunden auf 50°C. Zur Beseitigung der Peroxide setzte man Aktivkohle zu, rührte eine Stunde bei 50°C, saugte ab und engte das Filtrat im Vak. ein. Den Rückstand digerierte man mit wässrigem Ammoniak und Dichlormethan, saugte ab und wusch mit Wasser und Dichlormethan. man erhielt 3.4 g Rohprodukt, das man aus 200 ml Nitromethan mit Bleicherdebehandlung umkristallisierte. man erhielt 2.7 g (53 %) der Titelverbindung als farblose Kristalle mit dem Schmp. 235-236°C.

### Beispiel 22

### 5-(2-Formylaminomethyl-propan-2-yl)-2-(6-methoxy-pyridin-3-yl)benzimidazol

Zu einer Lösung von 3.5 g des in Beispiel 4 d dargestellten 5-(2-Formylaminomethyl-propan-2-yl)-2-(6-hydroxy-pyridin-3-yl)benzimidazols in 20 ml Wasser und 13.6 ml 1 N KOH in Ethanol gab man unter Wasserkühlung 0.85 ml Methyliodid. Nach 24 Stunden saugte man ab und reinigte säulenchromatographisch (Kieselgel, Laufmittel Dichlormethan: methanol. Ammoniak = 20:1). Entsprechende Fraktionen wurden vereinigt, das Laufmittel im Vak. entfernt und der Rückstand mit Ethanol/Wasser digeriert. Man saugte ab und erhielt 2.2 g (57 %) der Titelverbindung, der pro Mol noch 0.75 mol Wasser anhaftete, als farblose Kristalle, die sich an der Luft rötlich färbten, mit einem Schmp. von 217-221°C.

### Beispiel 23

Analog dem Beispiel 22 setzte man 4.8 g des in Beispiel 4 e dargestellten 5-(2-Formylaminomethyl-propan-2-yl)-2-(2-hydroxy-6-methyl-pyridin-3-yl)benzimidazols mit 1.5 ml Methyliodid um und erhielt nach säulenchromatographischer Reinigung 2.9 g 5-(2-Formylaminomethyl-propan-2-yl)-2-(2-methoxy-6-methyl-pyridin-3-yl)benzimidazol, welches man in heißem Dioxan löste, Essigester bis zur beginnenden Trübung zugab und nach Kristallisation absaugte. So erhielt man 2.2 g (48 %) der reinen Substanz in Form gelblicher Kristalle mit dem Schmp. 201-203°C.

### Beispiel 24

### 5-(2-Aminocarbonyl-propan-2-yl)-2-(4-pyridinyl)benzimidazol

3.7 g des in Beispiel 15 hergestellten 5-(2-Cyano-propan-2-yl)-2-(4-pyridinyl)benzimidazols rührte man in 40 ml 80 % Schwefelsäure drei Stunden bei 50°C, wobei die Substanz langsam in Lösung ging. Man goß auf Eis, stellte mit Ammoniak alkalisch, saugte ab und kristallisierte aus Ethanol um. Man erhielt 2.4 g (61 %) der Titelverbindung als farblose Kristalle mit Schmp. 274-276°C.

### Beispiel 25

### 5-[2-(Dimethylaminocarbontyl-aminomethyl)-propan-2-yl]-2-(4-pyridinyl)benzimidazol

Zu 0.25 g der in Beispiel 1 dargestellten Titelverbindung in 6 ml Dichlormethan gab man unter Eiskühlung 0.13 ml Triethylamin und 0.1 g N,N-Dimethylcarbamidsäurechlorid. Man rührte 20 h bei Raumtemperatur, entfernte das Lösungsmittel im Vak., digerierte mit Wasser, saugte ab und reinigte durch Filtration über eine kurze Kieselgelsäule (CH₂Cl₂ mit 5 % CH₃OH). Man brachte zur Trockene und kristallisierte den Rückstand aus Essigester. Man erhielt 0.15 g der Titelverbindung mit dem Schmp. 136-138°C.

### Beispiel 26

### 5-(2-Acetamidomethyl-propan-2-yl)-2-(4-methylphenyl)benzimidazol

Zu 4.4 g des in Beispiel 1 f hergestellten 4-(2-Acetamidomethyl-propan-2-yl)-1,2-diaminobenzols in 120 ml trockenem Dichlormethan gab man 3.3 ml Triethylamin und dann unter Eiskühlung 3.06 g 4-Methylbenzoesäurechlorid. Nach 3 h Rühren bei Raumtemperatur wurde das Lösungsmittel im Vak. entfernt. Man löste in Wasser, extrahierte dreimal mit Dichlormethan, trocknete die organische Phase über Natriumsulfat, filtrierte und entfernte das Lösungsmittel im Vakuum. Den Rückstand (6.7 g) löste man in Eisessig und rührte 3 h bei 70°C. Man entfernte das Lösungsmittel im Vak. und reinigte den Rückstand säulenchromatographisch (Kieselgel, CH₂Cl₂ mit 5 % CH₃OH). Man erhielt 1.55 g der Titelverbindung mit dem Schmp. 230-232°C.

### Beispiel 27

### 5-(2-Aminomethyl-propan-2-yl)-2-(4-methylphenyl)benzimidazol

1.45 g der in Beispiel 26 hergestellten Verbindung wurden 48 h in 35 ml Ethanol und 35 ml konz. Salzsäure unter Rückfluß zum Sieden erhitzt. Man entfernte das Lösungsmittel im Vakuum, löste in Wasser und neutralisierte mit 2 N Ammoniak. Die ausgefallene Substanz wurde abgesaugt. Man erhielt 1.1 g der Titelverbindung mit dem Schmp. 45-48°C.

### Beispiel 28

### 5-(2-Formylaminomethyl-propan-2-yl)-2-(4-methylphenyl)benzimidazol

Das in Beispiel 27 erhaltene Amin formylierte man analog Beispiel 2 und erhielt die Titelverbindung in 82 % Ausbeute mit dem Schmp. 193-195°C nach Umkristallisation aus Essigester.

### Beispiel 29

### 5-(2-Acetamidomethyl-propan-2-yl)-2-methylbenzimidazol

5.0 g des in Beispiel 1 e dargestellten N,Nʹ-Diacetyl-4-(2-aminomethyl-propan-2-yl)-2-nitroanilins in 250 ml Ethanol hydrierte man in Gegenwart von 0.5 g 10 % Palladium auf Kohle bei Raumtemperatur und Normaldruck. Nachdem 1.25 l Wasserstoff aufgenommen waren, filtrierte man und entfernte das Lösungsmittel im Vak.. Der Rückstand wurde in 250 ml Eisessig 3 h bei 70°C gerührt und das Lösungsmittel im Vak. entfernt, versetzte mit Essigester und ließ kristallisieren. Man erhielt 2.4 g der Titelverbindung mit dem Schmp. 198-201°C.

### Beispiel 30

### 5-(2-Aminomethylypropan-2-yl)-2-methylbenzimidazol

2.4 g der in Beispiel 29 hergestellten Verbindung wurde in 60 ml Ethanol und 60 ml konz. Salzsäure 24 h unter Rückfluß zum Sieden erhitzt. Das Lösungsmittel wurde im Vak. entfernt, der Rückstand mit 2 N Ammoniak neutralisiert, dreimal mit Dichlormethan extrahiert und die organsiche Phase uber Na₂SO₄ getrocknet. Nach Filtration und Eindampfen erhielt man 0.87 g der Titelverbindung als Öl.

### Beispiel 31

### 5-(2-Formylaminomethyl-propan-2-yl)-2-methylbenzimidazol

Analog dem Beispiel 2 erhielt man aus 0.87 g des in Beispiel 30 erhaltenen Amins 0.5 g der Titelverbindung mit dem Schmp. 57-60°C nach säulenchromatographischer Reinigung (Kieselgel, CH₂Cl₂ mit 2.5 % CH₃OH) und Ausrühren mit Ligroin.

### Beispiel 32

### 5-[2-(i-Propylcarbonylaminomethyl)-propan-2-yl]-2-(4-methoxyphenyl)benzimidazol

1.9 g der in Beispiel 6 erhaltenen Verbindung setzte man 200 ml Dichlormethan mit 2.5 ml Triethylamin und 0.82 Isobuttersäurechlorid bei Raumtemperatur um. Nach 10 h entfernte man das Lösungsmittel im Vak. und digerierte den Ruckstand mit Wasser und 2 N Ammoniak. Man extrahierte viermal mit Dichlormethan, trocknete uber Na₂SO₄, filtrierte und engte ein. Man setzte Essigester zu, destillierte einen Teil mit dem Dichlormethan ab, ließ kristallisieren und saugte ab. Man kristallisierte aus Essigester um und erhielt 0.6 g der Titelverbindung mit Schmp. 218-220°C.

### Beispiel 33

### 5-(2-Methylsulfonylaminomethyl-propan-2-yl)-2-(4-methoxyphenyl)benzimidazol

Aus dem in Beispiel 6 erhaltenen Hydrochlorid setzte man die Base frei, indem man die Substanz mit Dichlormethan und konz. Ammoniak rührte, bis alles in Lösung ging und dann die Dichlormethanphase eindampfte.

Zu 4.1 g des so erhaltenen 5-(2-Aminomethyl-propan-2-yl)-2-(4-methoxyphenyl)benzimidazols und 2.5 ml Hünig-Base in 100 ml Dichlormethan tropfte man unter Rühren und Kühlen mit Eiswasser 1.1 ml Mesylchlorid. Man rührte 30 min bei Raumtemperatur, versetzte mit Wasser und trennte die organische Phase ab. dampfte ein, löste den Rückstand (5.1 g) in wenig Essigester, rieb an, verdünnte mit Ether auf 80 ml, saugte ab und kristallisierte aus Essigester/Ether um und erhielt 4.0 g der Titelverbindung als farblose Kristalle mit dem Schmp. 146-148°C.

### Beispiel 34

### 5-(2-Methylsulfonylaminomethyl-propan-2-yl)-2-(2-methoxy-4-methylmercapto-phenyl)benzimidazol

In eine Lösung von 13 g des in Beispiel 1 f erhaltenen Diamins und 18 g des Bisulfit-Addukts von 2-Methoxy-4-methylmercapto-benzaldehyd in 500 ml Methanol leitete man bei Raumtemperatur 8 h Luft ein. Dann wurde eingedampft und säulenchromatographsich gereinigt (1.5 1 Kieselgel, Trichlormethan:Methanol:Eisessig = 10:1:0.2). Man erhielt 16 g 5-(2-Acetamidomethyl-propan-2-yl)-2-(2-methoxy-4-methylmercapto-phenyl)benzimidazol als bräunlich zähe Masse, welche man in 400 ml Ethanol und 100 ml konz. Salzsäure 3 Tage unter Rückfluß zum Sieden erhitzte. man dampfte ein, stellte den Rückstand mit 2 N NaOH alkalisch und extrahierte mit Dichlormethan. Man reinigte säulenchromatographisch (Kieselgel, Dichlormethan/methanol. NH₃ = 10:1), gewann 1 g Ausgangsmaterial zurück und erhielt 5.4 g 5-(2-Aminomethyl-propan-2-yl)-2-(2-methoxy-4-methylmercapto-phenyl)benzimidazol als bräunliche zähe Masse. 2,7 g davon mesylierte man analog dem Beispiel 33 und erhielt 3.1 g der Titelverbindung mit dem Schmp. 237-239°C als Dihydrat.

### Beispiel 35

### 5-(2-Methylsulfonylaminomethyl-propan-2-yl)-2-(2-methoxy-4-methylsulfinyl-phenyl)benzimidazol

1.4 g der in Beispiel 34 erhaltenen Verbindung in 28 ml Eisessig oxidierte man mit 0.4 ml 30 % Wasserstoffperoxid bei Raumtemperatur. Während 28 h ging die Substanz langsam in Lösung. Man verdünnte mit Wasser, pufferte mit konz. NH₃ und extrahierte mit Dichlormethan. Man reinigte säulenchromatographisch (800 ml Kieselgel, Dichlormethan/methanol. Ammoniak 20:1). Die reinen Fraktionen dampfte man ein, digerierte den Rückstand mit Essigester und erhielt 1.2 g der Titelverbindung mit dem Schmp. 126-128°C als Hydrat.

### Beispiel 36

### 5-(2-n-Propylsulfonylaminomethyl-propan-2-yl)-2-(4-pyridinyl)benzimidazol

Analog dem Beispiel 33 erhielt man aus 5.3 g 5-(2-Aminomethyl-propan-2-yl)-2-(4-pyridinyl)benzimidazol und 2.8 ml i-Propansulfonsäurechlorid die Titelverbindung in 42 % Ausbeute mit dem Schmp. 113-120°C nach Kristallisation aus Isopropanol. Der Verbindung haftet pro mol noch ein mol Isopropanol an.

### Beispiel 37

### 5-(2-i-Propansulfonylaminomethyl-propan-2-yl)-(4-pyridinyl)benzimidazol

Analog dem Beispiel 33 erhielt man aus 5.3 g 5-(2-Aminomethyl-propan-2-yl)-2-(4-pyridinyl)benzimidazol und 2.8 ml i-Propansulfonsäurechlorid die Titelverbindung in 5 % Ausbeute mit dem Schmp. 126-129°C nach Kristallisation aus Isopropanol. Der Verbindung haftet pro mol noch ein mol Isopropanol an.

### Beispiel 38

### 5-(2-Phenylsulfonylaminomethyl-propan-2-yl)-(4-pyridinyl)benzimidazol

Analog dem Beispiel 33 erhielt man aus 5.3 g 5-(2-Aminomethyl-propan-2-yl)-2-(4-pyridinyl)benzimidazol und 3.2 ml Phenylsulfonsäurechlorid die Titelverbindung in 50 % Ausbeute mit dem Schmp. 135-140°C nach Kristallisation aus Isopropanol. Der Verbindung haftet pro mol noch ein mol Isopropanol an.

### Beispiel 39

### 5-(2-Acetamidomethyl-propan-2-yl)-2-(4-imidazolyl)benzimidazol

4.40 g des in Beispiel 1 f hergestellten Diamins und 2.44 g Imidazol-4-carbonsäure erhitzte man unter Stickstoff in 50 ml Polyphosphorsäure 4.5 h auf 160°C. Man gab nochmals 2.44 g Imidazol-4-carbonsäure zu und erhitzte 6 h auf 160°C. Man arbeitete nach Abkühlen mit Eis/Wasser durch und stellte mit konz. Ammoniak auf pH = 9. Man dekan tierte, digerierte die zurückbleibende Schmiere mit Wasser, nahm den Rückstand in Methanol/Dichlormethan = 1:3 auf, trocknete uber Na₂SO₄ und destillierte zur Trockene. Den Rückstand (3.95 g) löste man in Ethanol, säuerte mit ethanolischer Salzsäure an und engte ein. Man erhielt 1.4 g der Titelverbindung als Hydrochlorid mit dem Schmp. 235-237°C.

### Beispiel 40

### 5-(2-Formylaminomethyl-propan-2-yl)-2-(4-imidazolyl)benzimidazol

1.2 g der in Beispiel 39 erhaltenen Verbindung erhitzte man in 24 ml Ethanol und 28 ml konz. Salzsäure unter Rückfluß zum Sieden und arbeitete analog Beispiel 1 g auf. Das erhaltene 5-(2-Aminomethyl-propan-2-yl)-2-(4-imidazolyl)benzimidazol formylierte man analog Beispiel 2 und erhielt 0.1 g der Titelverbindung als Hydrochlorid mit dem Schmp. 240-246°C nach Kristallisation aus ethanolischer Salzsäure.

### Beispiel 41

### 5-(2-Aminomethyl-propan-2-yl)-2-phenylbenzimidazol

4.4 g des in Beispiel 1 f hergestellten Diamins und 2 ml Benzaldehyd in 50 ml Ethanol und 5 ml Eisessig rührte man bei Raumtemperatur 18 Stunden. Man entfernte das Lösungsmittel im Vak., stellte mit 2 N Ammoniak alkalisch und extrahierte mit CH₂Cl₂, trocknete die organische Phase über Na₂SO₄ filtrierte und entfernte das Lösungsmittel im Vak.. Der Rückstand wurde in 100 ml Ethanol und 20 ml konz. Salzsäure 4 d unter Rückfluß zum Sieden erhitzt. Man entfernte das Lösungsmittel im Vak., stellte den Rückstand mit 2 N Ammoniak auf pH = 9 und erhielt 3 g der Titelverbindung mit dem Schmp. 185-187°C.

### Beispiel 42

5-(2-Aminomethyl-propan-2-yl)-2-(4-hydroxyphenyl)benzimidazol mit dem Schmp 178-182°C erhielt man analog Beispiel 41 durch Umsetzung mit 4-Hydroxybenzaldehyd.

### Beispiel 43

5-(2-Aminomethyl-propan-2-yl)-2-(2-furyl)benzimidazol erhielt man als Schaum analog dem Beispiel 41 durch Umsetzung mit Furfural.

### Beispiel 44

5-(2-Aminomethyl propan-2-yl)-2-(2-thienyl)benzimidazol erhielt man als Schaum analog dem Beispiel 41 durch Umsetzung mit Thiophen-2-aldehyd.

## Patentansprüche

1. 5-Alkylbenzimidazole der Formel I 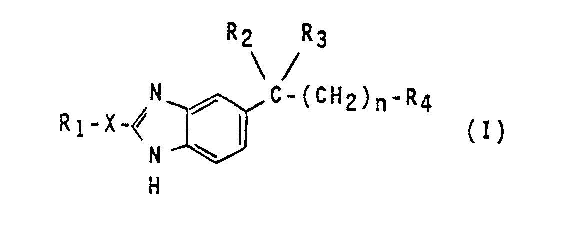 in welcher
R₁ einen Phenylring der allgemeinen Formel II darstellt, 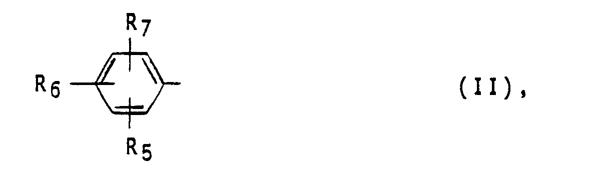
wobei R₅, R₆, R₇ gleich oder verschieden sein können und jeweils Wasserstoff, eine Alkansulfonyloxy-, Trifluormethansulfonyloxy-, Alkansulfonylamino-, Trifluormethansulfonylamino-, N-Alkyl-alkansulfonylamino-, N-Alkyltrifluormethansulfonylamino-, Alkylsulfenylmethyl-, Alkylsulfinylmethyl- oder Alkylsulfonylmethylgruppe, eine durch eine Hydroxy-, Alkoxy-, Amino-, Alkylamino- oder Dialkylaminogruppe substituierte Carbonylgruppe, eine durch eine Amino-, Alkylamino-, Dialkylamino- oder cyclische Iminogruppe substituierte Sulfonylgruppe, wobei eine Methylengruppe in 4-Stellung durch ein Schwefel- oder Sauerstoffatom ersetzt sein kann, eine Alkylcarbonylamino-, Aminocarbonylamino- oder Alkylaminocarbonylaminogruppe, eine Alkylmercapto-, Alkylsulfinyl- oder Alkylsulfonylgruppe, eine Nitro-, Halogen-, Amino-, Hydroxy-, Alkyl-, Alkoxy-, Alkenyloxy-, Alkinyloxy-, Cyanalkyloxy-, Carboxyalkoxy-, Alkoxycarbonylalkyloxy-, Dialkylamino-, 1-Imidazolyl-, Trifluormethyl- oder Cyangruppe sein können,
oder einen Naphthylrest oder einen gesättigten oder ungesättigten heterocyclischen Fünfring mit 1-4 Heteroatomen oder einen gesättigten oder ungesättigten heterocyclischen Sechsring mit 1-5 Heteroatomen darstellt, wobei die Heteroatome gleich oder verschieden sein können und Sauerstoff, Schwefel oder Stickstoff bedeuten und gewünschtenfalls an einem oder mehreren Stickstoffatomen ein Sauerstoffatom tragen können, und die Fünf- oder Sechsringe gegebenenfalls durch eine oder mehrere Alkyl-, Alkoxy-, Alkylmercapto-, Hydroxy-, Nitro-, Amino-, Halogen- oder Cyangruppen substituiert oder mit einem Phenylring zu einem Bicyclus kondensiert sein können,
oder für den Fall, daß X eine Bindung darstellt, R₁ neben den oben genannten Gruppen auch ein Wasserstoffatom, eine Alkyl-, Cycloalkyl-, Alkenyl-, Cycloalkenyl-, Alkinyl-, Halogenalkyl-, Alkoxyalkyl-, Carboxylalkyl-, Alkoxycarbonylalkyl-, Aminoalkyl-, eine Hydroxy-, Mercapto-, Amino-, Alkylmercapto-, Alkylcarbonylamino-, Formylamino-, Alkylsulfonylamino-, Formylaminoalkyl-, Alkoxycarbonylaminoalkyl- oder eine Alkylsulfonylaminoalkylgruppe bedeutet,
R₂ und R₃ gleich oder verschieden sein können und Wasserstoff oder Alkylgruppen bedeuten, oder R₂ und R₃ zusammen mit dem C-Atom an das sie gebunden sind einen carbocyclischen Ring darstellen,
R₄ eine Cyano-, Aminocarbonyl-, Alkylaminocarbonyl-, Dialkylaminocarbonyl-, Hydrazinocarbonyl- oder Aminogruppe bedeutet, wobei die Hydrazinocarbonyl- oder die Aminogruppe gewünschtenfalls durch eine Formyl-, Alkylcarbonyl-, Trifluormethylcarbonyl-, Alkylsulfonyl-, Trifluormethylsulfonylgruppe, Phenylsulfonyl-, Alkylaminocarbonyl- oder Dialkylaminocarbonylgruppe substituiert sein kann,
X eine Bindung, eine Alkylen-, die Vinylen-, die Iminogruppe -NH- oder die Carbonylaminogruppe -CONH- bedeutet und
n einen Wert zwischen 0 und 5 annehmen kann,
deren Tautomere und deren physiologisch verträgliche Salze anorganischer oder organischer säuren.

2. Verbindungen der Formel I gemäß Anspruch 1, 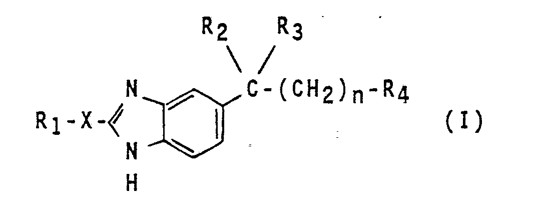 in welcher
R₁ den Phenylrest der allgemeinen Formel II bedeutet, in der
R₅ Wasserstoff, die Methansulfonyloxy-, Trifluormethansulfonyloxy-, Methansulfonylamino-, Trifluormethansulfonylamino-, Methansulfonyl-methylamino-, Trifluormethansulfonyl-methylamino-, Methylsulfenylmethyl-, Methylsulfinylmethyl-, Methylsulfonylmethyl-, Aminocarbonyl-, Aminosulfonyl-, Methylaminosulfonyl-, Dimethylaminosulfonyl-, Acetylamino-, Methylmercapto-, Methylsulfinyl-, Methylsulfonyl-, Hydroxy-, Methyl-, Methoxy-, Propargyloxy-, Cyanmethyloxy-, Methoxycarbonylmethyloxy-, Cyan-, Chlor-, Nitro-, Amino-, Dimethylamino-, Trifluormethyl- oder die 1-Imidazolylgruppe,
R₆ Wasserstoff, die Methyl-, Methoxy-, Dimethylamino- oder Chlorgruppe bedeutet,
R₇ Wasserstoff oder die Methoxygruppe ist
oder
R₁ den Pyrrol-, Furan-, Thiophen-, Pyrazol-, Imidazol-, Isothiazol-, Thiazol-, Oxazol-, Triazol-, Tetrazol-, Thiadiazol-, Isoxazol-, Oxadiazol-, Pyridin-, N-Oxy-pyridin-, Pyrazin-, N,N,-Dioxy-pyrazin, Pyrimidin-, N,Nʹ-Dioxypyrimidin-, Pyridazin-, Oxazin-, Thiazin-, Triazin- oder Tetrazinrest darstellt, sowie deren Methyl-, Ethyl-, Methoxy-, Ethoxy-, Methylmercapto-, Ethylmercapto- und chlorsubstituierten Derivate, oder in den Indol-, Indazol-, Chinolin-, Isochinolin-, Pyrrolidin-, Piperidin-, Morpholin-, Thiomorpholin- oder Naphthylrest bedeutet,
oder
R₁ für den Fall, daß X eine Bindung darstellt, neben den genannten Gruppen auch ein Wasserstoffatom, die Methyl-, Ethyl-, Propyl-, Butyl-, Pentyl-, Hexyl-, Propenyl-, Cyclopentenyl-, Cyclohexyl-, Trifluormethyl-, Hydroxy-, Mercapto-, Methylmercapto-, Amino-, Acetylamino-, Formylaminogruppe bedeutet,
R₂ und R₃ gleich sind und Methylgruppen bedeuten, oder
R₂ und R₃ zusammen einen Cyclopentanring bilden,
R₄ die Cyano-, Aminocarbonyl-, Amino-, Formylamino-, Acetylamino-, Isopropionylamino-, tert.-Butylcarbonylamino-, Trifluormethylcarbonylamino-, Methylsulfonylamino-, n-Propylsulfonylamino-, i-Propylsulfonylamino-, Trifluormethylsulfonylamino-, Phenylsulfonylamino-, Methylaminocarbonylamino- oder die Acetylaminocarbonylaminogruppe bedeutet,
X eine Bindung, die Methylengruppe, die Iminogruppe oder eine Carbonylaminogruppe darstellt,
n den Wert 0 oder 1 annimmt.

3. Verbindungen der Formel I gemäß Anspruch 1 oder 2, 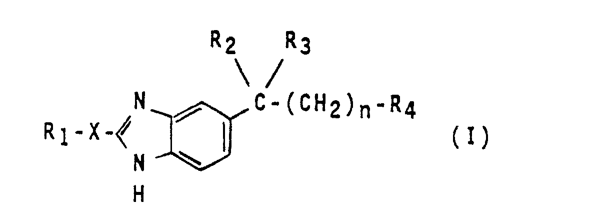 in welcher
R₁ Methyl, einen Piperidinyl-, Imidazolyl-, Furyl-, Thienyl- oder Chinolyl-Rest, einen gegebenenfalls ein- oder mehrfach substituierten Pyridyl-Rest oder einen gegebenenfalls ein- oder mehrfach durch Hydroxy, Methyl, Methoxy, Methylsulfenyl, Methylsulfinyl, Methylsulfonyl oder Halogen substituierten Phenylrest,
R₂ und R₃ jeweils eine Methylgruppe oder zusammen mit dem C-Atom, an das sie gebunden sind, einen Cyclopentyl-Ring darstellen,
R₄ eine Cyano-, Aminocarbonyl-, Amino-, Formylamino-, Acetylamino-, Isopropylcarbonylamino-, tert.Butylcarbonylamino, Dimethylaminocarbonylamino-, Methylsulfonylamino-, n-Propylsulfonylamino, Isopropylsulfonylamino- oder Phenylsulfonylamino-Gruppe,
X eine Bindung oder eine Methylengruppe und
n die Zahl 0 oder 1
bedeuten.

4. Verfahren zur Herstellung von 5-Alkylbenzimidazolen der Formel I 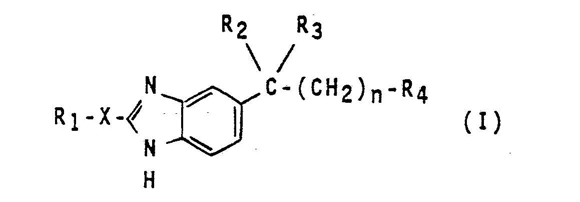 in welcher
R₁ einen Phenylring der allgemeinen Formel II darstellt, 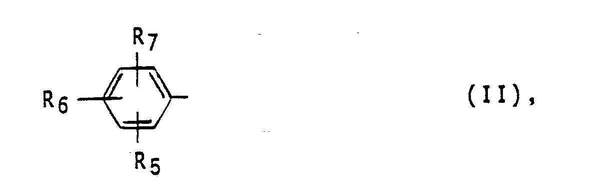
wobei R₅, R₆, R₇ gleich oder verschieden sein können und jeweils Wasserstoff, eine Alkansulfonyloxy-, Trifluormethansulfonyloxy-, Alkansulfonylamino-, Trifluormethansulfonylamino-, N-Alkyl-alkansulfonylamino-, N-Alkyltrifluormethansulfonylamino-, Alkylsulfenylmethyl-, Alkylsulfinylmethyl- oder Alkylsulfonylmethylgruppe, eine durch eine Hydroxy-, Alkoxy-, Amino-, Alkylamino- oder Dialkylaminogruppe substituierte Carbonylgruppe, eine durch eine Amino-, Alkylamino-, Dialkylamino- oder cyclische Iminogruppe substituierte Sulfonylgruppe, wobei eine Methylengruppe in 4-Stellung durch ein Schwefel- oder Sauerstoffatom ersetzt sein kann, eine Alkylcarbonylamino-, Aminocarbonylamino- oder Alkylaminocarbonylaminogruppe, eine Alkylmercapto-, Alkylsulfinyl- oder Alkylsulfonylgruppe, eine Nitro-, Halogen-, Amino-, Hydroxy-, Alkyl-, Alkoxy-, Alkenyloxy-, Alkinyloxy-, Cyanalkyloxy-, Carboxyalkoxy-, Alkoxycarbonylalkyloxy-, Dialkylamino-, 1-Imidazolyl-, Trifluormethyl- oder Cyangruppe sein können,
oder einen Naphthylrest oder einen gesättigten oder ungesättigten heterocyclischen Fünfring mit 1-4 Heteroatomen oder einen gesättigten oder ungesättigten heterocyclischen Sechsring mit 1-5 Heteroatomen darstellt, wobei die Heteroatome gleich oder verschieden sein können und Sauerstoff, Schwefel oder Stickstoff bedeuten und gewünschtenfalls an einem oder mehreren Stickstoffatomen ein Sauerstoffatom tragen können, und die Fünf- oder Sechsringe gegebenenfalls durch eine oder mehrere Alkyl-, Alkoxy-, Alkylmercapto-, Hydroxy-, Nitro-, Amino-, Halogen- oder Cyangruppen substituiert oder mit einem Phenylring zu einem Bicyclus kondensiert sein können,
oder für den Fall, daß X eine Bindung darstellt, R₁ neben den oben genannten Gruppen auch ein Wasserstoffatom, eine Alkyl-, Cycloalkyl-, Alkenyl-, Cycloalkenyl-, Alkinyl-, Halogenalkyl-, Alkoxyalkyl-, Carboxylalkyl-, Alkoxycarbonylalkyl-, Aminoalkyl-, eine Hydroxy-, Mercapto-, Amino-, Alkylmercapto-, Alkylcarbonylamino-, Formylamino-, Alkylsulfonylamino-, Formylaminoalkyl-, Alkoxycarbonylaminoalkyl- oder eine Alkylsulfonylaminoalkylgruppe bedeutet,
R₂ und R₃ gleich oder verschieden sein können und Wasserstoff oder Alkylgruppen bedeuten, oder R₂ und R₃ zusammen mit dem C-Atom an das sie gebunden sind einen carbocyclischen Ring darstellen,
R₄ eine Cyano-, Aminocarbonyl-, Alkylaminocarbonyl-, Dialkylaminocarbonyl-, Hydrazinocarbonyl- oder Aminogruppe bedeutet, wobei die Hydrazinocarbonyl- oder die Aminogruppe gewünschtenfalls durch eine Formyl-, Alkylcarbonyl-, Trifluormethylcarbonyl-, Alkylsulfonyl-, Trifluormethylsulfonylgruppe, Phenylsulfonyl-, Alkylaminocarbonyl- oder Dialkylaminocarbonylgruppe substituiert sein kann,
X eine Bindung, eine Alkylen-, die Vinylen-, die Iminogruppe -NH- oder die Carbonylamonogruppe -CONH- bedeutet und
n einen Wert zwischen 0 und 5 annehmen kann,
deren Tautomere und deren physiologisch verträglichen Salze anorganischer oder organischer Säuren,
dadurch gekennzeichnet, daß man in dem Fall, daß R₁ die angegebene Bedeutung hat mit der Ausnahme, daß R₁ keine Amino-, Hydroxy- oder Mercaptogruppe darstellen darf, wenn X eine Bindung bedeutet,
a) eine Verbindung der Formel V 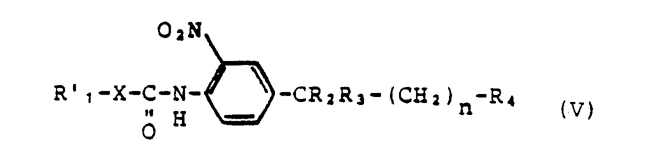 in der R₂, R₃, R₄, X und n die angegebenen Bedeutungen haben und R₁ die vorstehend angegebene Bedeutung von R₁, hat, reduziert und cyclisiert.
oder
b) eine Verbindung der Formel VI 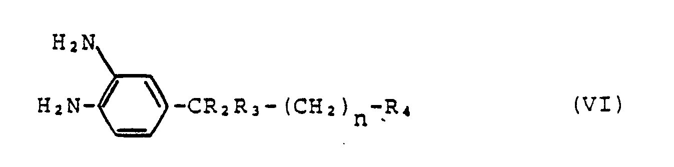 in der R₂, R₃, R₄ und n die oben angegebenen Bedeutungen haben, mit einer Verbindung der Formel VII
R'₁-X- 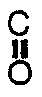 -Y (VII)
in der R₁, und X die vorstehend angegebenen Bedeutungen besitzen und Y entweder ein Wasserstoffatom, die Hydroxygrupe oder eine leicht abspaltbare Gruppe bedeutet, umsetzt
oder
c) für den Fall, daß X einen Valenzstrich und R₁ eine Amino-, Hydroxy- oder Mercaptogruppe bedeuten,
eine Verbindung der Formel VI 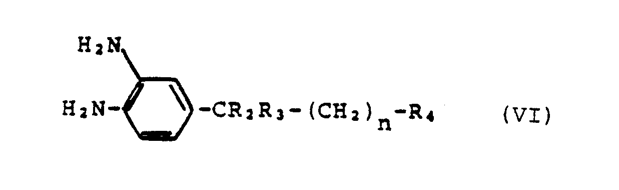
in der
R₂, R₃, R₄ und n die angegebenen Bedeutungen haben und R₁ die vorstehend angegführte Bedeutung von R₁ besitzt, mit einer Carbonyl-, Thiocarbonyl- oder Iminogruppe enthaltenen Verbindung umsetzt
und anschließend die erhaltenen Verbindungen der Formel I oder deren Tautomere gewunschtenfalls in eine andere Verbindung der Formel I umwandelt und/oder in ein physiologisch verträgliches Salz einer anorganisch oder organischen Säure überführt.

5. Verfahren zur Herstellung von Verbindungen der Formel I gemäß Anspruch 4,
in welcher
R₁ den Phenylrest der allgemeinen Formel II bedeutet, in der
R₅ Wasserstoff, die Methansulfonyloxy-, Trifluormethansulfonyloxy-, Methansulfonylamino-, Trifluormethansulfonylamino-, Methansulfonyl-methylamino-, Trifluormethansulfonyl-methylamino-, Methylsulfenylmethyl-, Methylsulfinylmethyl-, Methylsulfonylmethyl-, Aminocarbonyl-, Aminosulfonyl-, Methylaminosulfonyl-, Dimethylaminosulfonyl-, Acetylamino-, Methylmercapto-, Methylsulfinyl-, Methylsulfonyl-, Hydroxy-, Methyl-, Methoxy-, Propargyloxy-, Cyanmethyloxy-, Methoxycarbonylmethyloxy-, Cyan-, Chlor-, Nitro-, Amino-, Dimethylamino-, Trifluormethyl- oder die 1-Imidazolylgruppe,
R₆ Wasserstoff, die Methyl-, Methoxy-, Dimethylamino- oder Chlorgruppe bedeutet,
R₇ Wasserstoff oder die Methoxygruppe ist
oder
R₁ den Pyrrol-, Furan-, Thiophen-, Pyrazol-, Imidazol-, Isothiazol-, Thiazol-, Oxazol-, Triazol-, Tetrazol-, Thiadiazol-, Isoxazol-, Oxadiazol-, Pyridin-, N-Oxy-pyridin-, Pyrazin-, N,Nʹ-Dioxy-pyrazin, Pyrimidin-, N,Nʹ-Dioxypyrimidin-, Pyridazin-, Oxazin-, Thiazin-, Triazin-oder Tetrazinrest darstellt, sowie deren Methyl-, Ethyl-, Methoxy-, Ethoxy-, Methylmercapto-, Ethylmercapto- und chlorsubstituierten Derivate, oder in den Indol-, Indazol-, Chinolin-, Isochinolin-, Pyrrolidin-, Piperidin-, Morpholin-, Thiomorpholin- oder Naphthylrest bedeutet,
oder
R₁ für den Fall, daß X eine Bindung darstellt, neben den genannten Gruppen auch ein Wasserstoffatom, die Methyl-, Ethyl-, Propyl-, Butyl-, Pentyl-, Hexyl-, Propenyl-, Cyclopentenyl-, Cyclohexyl-, Trifluormethyl-, Hydroxy-, Mercapto-, Methylmercapto-, Amino-, Acetylamino-, Formylaminogruppe bedeutet,
R₂ und R₃ gleich sind und Methylgruppen bedeuten, oder
R₂ und R₃ zusammen einen Cyclopentanring bilden,
R₄ die Cyano-, Aminocarbonyl-, Amino-, Formylamino-, Acetylamino-, Isopropionylamino-, tert.-Butylcarbonylamino-, Trifluormethylcarbonylamino-, Methylsulfonylamino-, n-Propylsulfonylamino-, i-Propylsulfonylamino-, Trifluormethylsulfonylamino-, Phenylsulfonylamino-, Methylaminocarbonylamino- oder die Acetylaminocarbonylaminogruppe bedeutet,
X eine Bindung, die Methylengruppe, die Iminogruppe oder eine Carbonylaminogruppe darstellt,
n den Wert 0 oder 1 annimmt.

6. Verfahren zur Herstellung von Verbindungen der Formel I gemäß Anspruch 4, 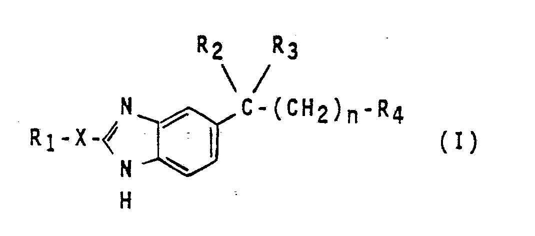
in welcher
R₁ Methyl, einen Piperidinyl-, Imidazolyl-, Furyl-, Thienyl- oder Chinolyl-Rest, einen gegebenenfalls ein-oder mehrfach substituierten Pyridyl-Rest oder einen gegebenenfalls ein- oder mehrfach durch Hydroxy, Methyl, Methoxy, Methylsulfenyl, Methylsulfinyl, Methylsulfonyl oder Halogen substituierten Phenylrest,
R₂ und R₃ jeweils eine Methylgruppe oder zusammen mit dem C-Atom, an das sie gebunden sind, einen Cyclopentyl-Ring darstellen,
R₄ eine Cyano-, Aminocarbonyl-, Amino-, Formylamino-, Acetylamino-, Isopropylcarbonylamino-, Dimethylaminocarbonylamino-, Methylsulfonylamino-, n-Propylsulfonylamino, Isopropylsulfonylamino- oder Phenylsulfonylamino-Gruppe,
X eine Bindung oder eine Methylengruppe und
n die Zahl 0 oder 1
bedeuten.

7. Arzneimittel, enthaltend mindestens eine Verbindung gemäß Anspruch 1, 2 oder 3 neben üblichen Träger- und Hilfsstoffen.

8. Verwendung von Verbindungen gemäß Anspruch 1 zur Herstellung von Arzneimitteln zur Behandlung von Herz- und Kreislauferkrankungen.

9. Verwendung von Verbindundungen gemäß Anspruch 1 zur Behandlung von Herz- und Kreislauferkrankungen.
